# EUROPÄISCHE PATENTSCHRIFT

(11) **EP 1 427 699 B1**
(45) Veröffentlichungstag und Bekanntmachung des Hinweises auf die Patenterteilung: **03.08.2011**
(21) Anmeldenummer: 02797547.3
(22) Anmeldetag: 21.08.2002
(51) Int. Cl.: C07C 311/51, C07D 307/66, A01N 41/06, A01N 43/653, C07D 249/08

(54) **SELEKTIVE HERBIZIDE AUF BASIS VON SUBSTITUIERTEN ARYLSULFONYLAMINOCARBONYLTRIAZOLINONEN UND SAFENERN**
SELECTIVE HERBICIDES BASED ON SUBSTITUTED ARYLSULFONYLAMINOCARBONYL TRIAZOLINONES AND SAFENERS
HERBICIDES SELECTIFS A BASE D'ARYLSULFONYLAMINOCARBONYL-TRIAZOLINONES ET PHYTOPROTECTEURS

(30) Priorität: 03.09.2001 DE 10143083
(43) Veröffentlichungstag der Anmeldung: 16.06.2004
(73) Patentinhaber: Arysta LifeScience North America, LLC, Cary, NC 27513 (US)
(72) Erfinder: FEUCHT, Dieter, 65760 Eschborn (DE); DAHMEN, Peter, 41470 Neuss (DE); DREWES, Mark, Wilhelm, 40764 Langenfeld (DE); PONTZEN, Rolf, 42799 Leichlingen (DE); MÜLLER, Klaus-Helmut, 40593 Düsseldorf (DE); SCHWARZ, Hans-Georg, 40764 Langenfeld (DE)
(74) Vertreter: Vossius & Partner
(86) Internationale Anmeldenummer: PCT/EP2002/009329
(87) Internationale Veröffentlichungsnummer: WO 2003/020692

(56) Entgegenhaltungen:
- EP-A- 0 931 456
- US-B1- 6 235 680

## Beschreibung

Die Erfindung betrifft neue selektiv-herbizide Wirkstoffkombinationen, die substituierte Arylsulfonylaminocarbonyl-triazolinone und/oder deren Salze einerseits und zumindest eine die Kulturpflanzen-Verträglichkeit verbessernde Verbindung andererseits enthalten und mit besonders gutem Erfolg zur selektiven Unkrautbekämpfung in verschiedenen Nutzpflanzenkulturen verwendet werden können.

Substituierte Arylsulfonylaminocarbonyl-triazolinone sind bereits als wirksame Herbizide bekannt (vgl. EP-A-341489, EP-A-422469, EP-A-425948, EP-A-431291, EP-A-507171, EP-A-534266, WO-A-96/11188, WO-A-96/27590, WO-A-96/27591, WO-A-97/03056). Die Wirkung dieser Verbindungen und/oder ihre Verträglichkeit gegenüber Kulturpflanzen sind jedoch nicht unter allen Bedingungen ganz zufriedenstellend.

Weiter sind Kombinationen aus substituierten Arylsulfonylaminocarbonyl-triazolinonen und anderen herbizid wirksamen Verbindungen zum Erzielen einer synergistischen Wirkung bekannt geworden (vgl. WO-A-98/12923). Auch Kombinationen aus substituierten Arylsulfonylaminocarbonyl-triazolinonen und Safenern sind bereits bekannt (vgl. DE-A-19940859, DE-A-19940860, US-A-6162762). Auch bei diesen Kombinationsprodukten sind jedoch die Anwendungseigenschaften nicht unter allen Bedingungen ganz befriedigend. Ferner sind auch Kombinationen aus substituierten Arylsulfonylaminocarbonyl-triazolinonen und weiteren herbizid wirksamen Verbindungen, gegebenenfalls unter zusätzlichem Einsatz von Safenern, Gegenstand einer vorgängigen, jedoch nicht vorveröffentlichten Patentanmeldung (vgl. DE-A-10031825 vom 30.06.2000).

Überraschenderweise wurde nun gefunden, dass bestimmte substituierte Arylsulfonylaminocarbonyl-triazolinone bei gemeinsamer Anwendung mit den im weiteren beschriebenen, die Kulturpflanzen-Verträglichkeit verbessernden Verbindungen (Safenem/Antidots) ausgesprochen gut die Schädigung der Kulturpflanzen verhindern und besonders vorteilhaft als breit wirksame Kombinationspräparate zur selektiven Bekämpfung von Unkräutern in Nutzpflanzenkulturen, wie z.B. in Getreide, Mais und Reis verwendet werden können.

Gegenstand der Erfindung sind selektiv-herbizide Mittel, gekennzeichnet durch einen wirksamen Gehalt an einer Wirkstoffkombination umfassend
(a) ein oder mehrere substituierte Arylsulfonylaminocarbonyl-triazolinone der Formel (I) 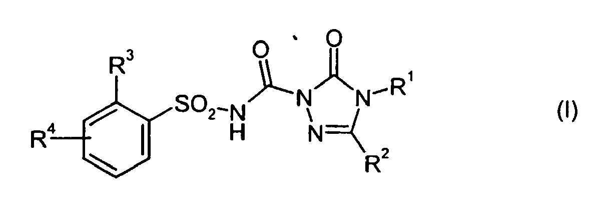 in welcher
   - R¹: für Wasserstoff, Hydroxy, Amino, C₂-C₆-Alkylidenamino, für jeweils gege- benenfalls durch Cyano, Halogen oder C₁-C₄-Alkoxy substituiertes Alkyl, Alkenyl, Alkinyl, Alkoxy, Alkenyloxy, Alkylamino oder Dialkylamino mit jeweils bis zu 6 Kohlenstoffatomen, für jeweils gegebenenfalls durch Cyano, Halogen oder C₁-C₄-Alkyl substituiertes Cycloalkyl, Cycloalkylalkyl oder Cycloalkylamino mit jeweils 3 bis 6 Kohlenstoffatomen in den Cycloalkyl- gruppen und gegebenenfalls 1 bis 4 Kohlenstoffatomen im Alkylteil, oder für jeweils gegebenenfalls durch Cyano, Nitro, Halogen, C₁-C₄-Alkyl, C₁-C₄- Halogenalkyl, C₁-C₄-Alkoxy oder C₁-C₄-Halogenalkoxy substituiertes Phenyl oder Phenyl-C₁-C₄-alkyl steht,
   - R²: für Wasserstoff, Hydroxy, Mercapto, Amino, Cyano, Halogen, für jeweils ge- gebenenfalls durch Cyano, Halogen oder C₁-C₄-Alkoxy substituiertes Alkyl, Alkoxy, Alkylthio, Alkylamino, Dialkylamino, Alkenyl, Alkinyl, Alkenyl- oxy, Alkinyloxy, Alkenylthio, Alkinylthio, Alkenylamino oder Alkinylamino mit jeweils bis zu 6 Kohlenstoffatomen, für jeweils gegebenenfalls durch Cyano, Halogen oder C₁-C₄-Alkyl substituiertes Cycloalkyl, Cycloalkyloxy, Cycloalkylthio, Cycloalkylamino, Cycloalkylalkyl, Cycloalkylalkoxy, Cyclo- alkylalkylthio oder Cycloalkylalkylamino mit jeweils 3 bis 6 Kohlenstoff- atomen in den Cycloalkylgruppen und gegebenenfalls 1 bis 4 Kohlenstoff- atomen im Alkylteil, oder für jeweils gegebenenfalls durch Cyano, Nitro, Halogen, C₁-C₄-Alkyl, C₁-C₄-Halogenalkyl, C1-C₄-Alkoxy oder C₁-C₄- Halogenalkoxy substituiertes Phenyl, Phenoxy, Phenylthio, Phenylamino oder Phenyl-C₁-C₄-alkyl steht,
   - R³: für Nitro, Cyano, Halogen, für jeweils gegebenenfalls durch Cyano, Halogen oder C₁-C₄-Alkoxy substituiertes Alkyl, Alkylcarbonyl, Alkoxy, Alkoxy- carbonyl, Alkylthio, Alkylsulfinyl, Alkylsulfonyl, Alkylamino, Alkenyl, Alkenyloxy, Alkenylthio, Alkenylamino, Alkinyl, Alkinyloxy, Alkinylthio mit jeweils bis zu 6 Kohlenstoffatomen, für jeweils gegebenenfalls durch Cyano, Halogen oder C₁-C₄-Alkyl substituiertes Cycloalkyl, Cycloalkyloxy, Cycloalkylthio, Cycloalkylamino mit jeweils 3 bis 6 Kohlenstoffatomen in den Cycloalkylgruppen, oder für jeweils gegebenenfalls durch Cyano, Nitro, Halogen, C₁-C₄-Alkyl, C₁-C₄-Halogenalkyl, C₁-C₄-Alkoxy oder C₁-C₄- Halogenalkoxy substituiertes Phenyl, Phenoxy, Phenylthio, Phenylsulfinyl, Phenylsulfonyl oder Phenylamino steht, und
   - R⁴: für Wasserstoff, Nitro, Cyano, Halogen, für jeweils gegebenenfalls durch Cyano, Halogen oder C₁-C₄-Alkoxy substituiertes Alkyl, Alkylcarbonyl, Alkoxy, Alkoxycarbonyl, Alkylthio, Alkylsulfinyl, Alkylsulfonyl, Alkyl- amino, Alkenyl, Alkenyloxy, Alkenylthio, Alkenylamino, Alkinyl, Alkinyl- oxy oder Alkinylthio mit jeweils bis zu 6 Kohlenstoffatomen, für jeweils ge- gebenenfalls durch Cyano, Halogen oder C₁-C₄-Alkyl substituiertes Cyclo- alkyl, Cycloalkyloxy, Cycloalkylthio, Cycloalkylamino mit jeweils 3 bis 6 Kohlenstoffatomen in den Cycloalkylgruppen, oder für jeweils gegebenen- falls durch Cyano, Nitro, Halogen, C₁-C₄-Alkyl, C₁-C₄-Halogenalkyl, C₁- C₄-Alkoxy oder C₁-C₄-Halogenalkoxy substituiertes Phenyl, Phenoxy, Phenylthio, Phenylsulfinyl, Phenylsulfonyl oder Phenylamino steht,
   - einschließlich aller möglichen tautomeren Formen der Verbindungen der allgemeinen Formel (I) sowie der aus Verbindungen der allgemeinen Formel (I) und basischen Verbindungen gebildeten Salze -
   ("wirksame Verbindungen der Gruppe 1")
   und
(b) zumindest eine die Kulturpflanzen-Verträglichkeit verbessernde Verbindung aus der folgenden Gruppe von Verbindungen der allgemeinen Formel (II) 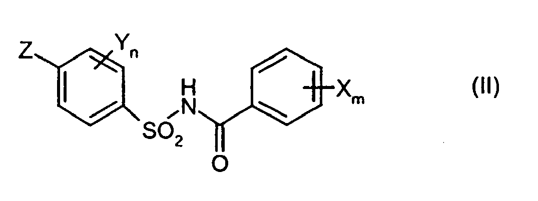 in welcher
   - m: für die Zahlen 0 bis 5 steht,
   - n: für die Zahlen 0 bis 4 steht,
   - X: für Nitro, Cyano, Carboxy, Carbamoyl, Formyl, Sulfamoyl, Hydroxy, Amino, Halogen, oder für jeweils gegebenenfalls durch Cyano, Halogen oder C₁-C₄-Alkoxy substituiertes Alkyl, Alkoxy, Alkoxy- carbonyl, Alkylthio, Alkylsulfinyl, Alkylsulfonyl, Alkylamino, Alkyl- aminocarbonyl mit jeweils 1 bis 6 Kohlenstoffatomen in den Alkyl- gruppen, oder für Dialkylamino, Dialkylaminocarbonyl oder Dialkyl- aminosulfonyl mit jeweils 1 bis 6 Kohlenstoffatomen in den Alkyl- gruppen steht,
   - Y: für Nitro, Cyano, Carboxy, Carbamoyl, Formyl, Sulfamoyl, Hydroxy, Amino, Halogen, oder für jeweils gegebenenfalls durch Cyano, Halogen oder C₁-C₄-Alkoxy substituiertes Alkyl oder Alkoxy mit jeweils 1 bis 6 Kohlenstoffatomen steht, und
   - Z: für die nachstehende Gruppierung steht

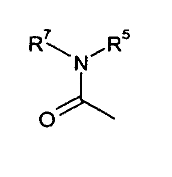

   - worin R⁵: für Wasserstoff, für gegebenenfalls durch Cyano, Halogen oder C₁-C₄-Alkoxy substituiertes Alkyl mit 1 bis 6 Kohlenstoff- atomen, für jeweils gegebenenfalls durch Halogen substitu- iertes Alkenyl oder Alkinyl mit jeweils 3 bis 6 Kohlenstoff- atomen, oder für jeweils gegebenenfalls durch Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil steht,

   - R⁷: für Wasserstoff, für gegebenenfalls durch Cyano, Halogen oder C₁-C₄-Alkoxy substituiertes Alkyl mit 1 bis 6 Kohlenstoff- atomen, für Alkoxy mit 1 bis 6 Kohlenstoffatomen, für jeweils gegebenenfalls durch Halogen substituiertes Alkenyl oder Alkinyl mit jeweils 3 bis 6 Kohlenstoffatomen, für Alkenyloxy mit 3 bis 6 Kohlenstoffatomen, für jeweils gegebenenfalls durch Halogen oder C₁-C₄-Alkyl substituiertes Cycloalkyl oder Cycloalkylalkyl mit 3 bis 6 Kohlenstoffatomen in der Cyclo- alkylgruppe und gegebenenfalls 1 bis 4 Kohlenstoffatomen im Alkylteil, oder für jeweils gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil steht, oder zusammen mit R⁵ für jeweils gegebenenfalls durch C₁-C₄-Alkyl substituiertes Alkandiyl mit 2 bis 6 Kohlenstoff- atomen oder Oxaalkandiyl mit 2 bis 5 Kohlenstoffatomen steht
("wirksame Verbindungen der Gruppe 2").

In den Definitionen sind die Kohlenwasserstoffketten, wie in Alkyl oder Alkandiyl - auch in Verbindung mit Heteroatomen, wie in Alkoxy - jeweils geradkettig oder verzweigt.

Bevorzugte Bedeutungen der oben in Zusammenhang mit der allgemeinen Formel (I) aufgeführten Gruppen werden im Folgenden definiert.
- R¹: steht bevorzugt für Wasserstoff, Amino, Propylidenamino, Butylidenamino, für jeweils gegebenenfalls durch Cyano, Fluor, Chlor, Methoxy oder Ethoxy substituiertes Methyl, Ethyl, n- oder i-Propyl, n-, i-, s- oder t-Butyl, Ethenyl, Propenyl, Butenyl, Ethinyl, Propinyl, Butinyl, Methoxy, Ethoxy, n- oder i- Propoxy, n-, i-, s- oder t-Butoxy, Propenyloxy, Butenyloxy, Methylamino, Ethylamino, n- oder i-Propylamino, n-, i-, s- oder t-Butylamino, Dimethyl- amino oder Diethylamino, für jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclopentylmethyl, Cyclohexylmethyl, Cyclopropylamino, Cyclobutylamino, Cyclopentylamino oder Cyclohexylamino, oder für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, Difluormethoxy oder Trifluormethoxy substituiertes Phenyl oder Benzyl.
- R²: steht bevorzugt für Wasserstoff, Hydroxy, Mercapto, Amino, Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t- Butyl, Methoxy, Ethoxy, n- oder i-Propoxy, n-, i-, s- oder t-Butoxy, Methyl- thio, Ethylthio, n- oder i-Propylthio, n-, i-, s- oder t-Butylthio, Methylamino, Ethylamino, n- oder i-Propylamino, n-, i-, s- oder t-Butylamino, Dimethyl- amino, Diethylamino, Ethenyl, Propenyl, Butenyl, Ethinyl, Propinyl, Butinyl, Propenyloxy, Butenyloxy, Propihyloxy, Butinyloxy, Propenylthio, Butenyl- thio, Propinylthio, Butinylthio, Propenylamino, Butenylamino, Propinyl- amino oder Butinylamino, für jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclo- pentylthio, Cyclohexylthio, Cyclopropylamino, Cyclobutylamino, Cyclo- pentylamino, Cyclohexylamino, Cyclopropylmethyl, Cyclobutylmethyl, Cyclopentylmethyl, Cyclohexylmethyl, Cyclopropylmethoxy, Cyclobutyl- methoxy, Cyclopentylmethoxy, Cyclohexylmethoxy, Cyclopropylmethylthio, Cyclobutylmethylthio, Cyclopentylmethylthio, Cyclohexylmethylthio, Cyclo- propylmethylamino, Cyclobutylmethylamino, Cyclopentylmethylamino oder Cyclohexylmethylamino, oder für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, Difluormethoxy oder Trifluormethoxy sub- stituiertes Phenyl, Phenoxy, Phenylthio, Phenylamino oder Benzyl.
- R³: steht bevorzugt für Nitro, Cyano, Fluor, Chlor, Brom, für jeweils gegebenen- falls durch Cyano, Fluor, Chlor, Methoxy oder Ethoxy substituiertes Methyl, Ethyl, n- oder i-Propyl, n-, i-, s- oder t-Butyl, Acetyl, Propionyl, n- oder i- Butyroyl, Methoxy, Ethoxy, n- oder i-Propoxy, n-, i-, s- oder t-Butoxy, Methoxycarbonyl, Ethoxycarbonyl, n- oder i-Propoxycarbonyl, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Ethenyl, Propenyl, Butenyl, Propenyloxy, Butenyloxy, Propenylthio, Butenylthio, Propenylamino, Butenylamino, Ethinyl, Propinyl, Butinyl, Propinyloxy, Butinyloxy, Propinylthio oder Butinylthio, für jeweils gegebenenfalls durch Cyano, Fluor, Chlor, Brom, Methyl, Ethyl, n- oder i- Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclo- propylthio, Cyclobutylthio, Cyclopentylthio, Cyclohexylthio, Cyclopropyl- amino, Cyclobutylamino, Cyclopentylamino oder Cyclohexylamino, oder für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy oder Tri- fluormethoxy substituiertes Phenyl, Phenoxy, Phenylthio, Phenylsulfinyl, Phenylsulfonyl oder Phenylamino.
- R⁴: steht bevorzugt für Wasserstoff, Nitro, Cyano, Fluor, Chlor, Brom, für jeweils gegebenenfalls durch Cyano, Fluor, Chlor, Methoxy oder Ethoxy sub- stituiertes Methyl, Ethyl, n- oder i-Propyl, n-, i-, s- oder t-Butyl, Acetyl, Propionyl, n- oder i-Butyroyl, Methoxy, Ethoxy, n- oder i-Propoxy, n-, i-, s- oder t-Butoxy, Methoxycarbonyl, Ethoxycarbonyl, n- oder i-Propoxy- carbonyl, Methylthio, Ethylthio, n- oder i-Propylthio, n-, i-, s- oder t-Butyl- thio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i- Propylamino, n-, i-, s- oder t-Butylamino, Ethenyl, Propenyl, Butenyl, Propenyloxy, Butenyloxy, Propenylthio, Butenylthio, Propenylamino, Butenylamino, Ethinyl, Propinyl, Butinyl, Propinyloxy, Butinyloxy, Propinylthio oder Butinylthio, für jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclo- pentylthio, Cyclohexylthio, Cyclopropylamino, Cyclobutylamino, Cyclo- pentylamino oder Cyclohexylamino, oder für jeweils gegebenenfalls durch Cyano, Nitro, Fluor, Chlor, Brom, Methyl, Ethyl, n- oder i-Propyl, Trifluor- methyl, Methoxy, Ethoxy, n- oder i-Propoxy, n-, i-, s- oder t-Butoxy, Difluor- methoxy oder Trifluormethoxy substituiertes Phenyl, Phenoxy, Phenylthio, Phenylsulfinyl, Phenylsulfonyl oder Phenylamino.
- R¹: steht besonders bevorzugt für Wasserstoff, Amino, für jeweils gegebenenfalls durch Fluor, Chlor, Methoxy oder Ethoxy substituiertes Methyl, Ethyl, Ethenyl, Propenyl, Ethinyl, Propinyl, Methoxy, Ethoxy, n- oder i-Propoxy, Methylamino oder Ethylamino, für Dimethylamino, oder für gegebenenfalls durch Fluor, Chlor oderMethyl substituiertes Cyclopropyl.
- R²: steht besonders bevorzugt für Wasserstoff, Chlor, Brom, für jeweils gegebe- nenfalls durch Fluor, Chlor, Methoxy oder Ethoxy substituiertes Methyl, Ethyl, n- oder i-Propyl, n-, i- oder s-Butyl, Methoxy, Ethoxy, n- oder i- Propoxy, Methylthio, Ethylthio, n- oder i-Propylthio, Methylamino, Ethyl- amino, n- oder i-Propylamino, Dimethylamino, Diethylamino, Ethenyl, Propenyl, Butenyl, Ethinyl, Propinyl, Butinyl, Propenyloxy, Butenyloxy, Propinyloxy, Butinyloxy, Propenylthio, Butenylthio, Propinylthio, Butinyl- thio, Propenylamino, Butenylamino, Propinylamino oder Butinylamino, oder für jeweils gegebenenfalls durch Fluor, Chlor oder Methyl substituiertes Cyclopropyl, Cyclobutyl, Cyclopentyl, Cyclohexyl, Cyclopropyloxy, Cyclo- butyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutyl- methoxy, Cyclopentylmethoxy, Cyclohexylmethoxy, Cyclopropylmethylthio, Cyclobutylmethylthio, Cyclopentylmethylthio, Cyclohexylmethylthio, Cyclo- propylmethylamino, Cyclobutylmethylamino, Cyclopentylmethylamino oder Cyclohexylmethylamino.
- R³: steht besonders bevorzugt für Nitro, Cyano, Fluor, Chlor, Brom, für jeweils gegebenenfalls durch Fluor, Chlor, Methoxy oder Ethoxy substituiertes Methyl, Ethyl, n- oder i-Propyl, Methoxy, Ethoxy, n- oder i-Propoxy, Methoxycarbonyl, Ethoxycarbonyl, n- oder i-Propoxycarbonyl, Methylthio, Ethylthio, n- oder i-Propylthio, Methylsulfinyl, Ethylsulfinyl, Methylsulfonyl oder Ethylsulfonyl, für jeweils gegebenenfalls durch Fluor, Chlor oder Methyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclo- propylthio, Cyclobutylthio, Cyclopentylthio oder Cyclohexylthio, oder für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substi- tuiertes Phenyl, Phenoxy, Phenylthio, Phenylsulfinyl oder Phenylsulfonyl.
- R⁴: steht besonders bevorzugt für Wasserstoff, Nitro, Cyano, Fluor, Chlor, Brom, oder für jeweils gegebenenfalls durch Fluor, Chlor, Methoxy oder Ethoxy substituiertes Methyl, Ethyl, n- oder i-Propyl, Methoxy, Ethoxy, n- oder i- Propoxy, Methoxycarbonyl, Ethoxycarbonyl, n- oder i-Propoxycarbonyl, Methylthio, Ethylthio, n- oder i-Propylthio, Methylsulfinyl, Ethylsulfinyl, Methylsulfonyl oder Ethylsulfonyl.
- R¹: steht ganz besonders bevorzugt für Wasserstoff, für jeweils gegebenenfalls durch Fluor, Chlor, Methoxy oder Ethoxy substituiertes Methyl, Ethyl, Ethenyl, Methoxy, Ethoxy, Methylamino oder Ethylamino, für Dimethyl- amino, oder für gegebenenfalls durch Fluor, Chlor oder Methyl substituiertes Cyclopropyl.
- R²: steht ganz besonders bevorzugt für Wasserstoff, Chlor, Brom, für jeweils ge- gebenenfalls durch Fluor, Chlor, Methoxy oder Ethoxy substituiertes Methyl, Ethyl, n- oder i-Propyl, n-, i- oder s-Butyl, Methoxy, Ethoxy, n- oder i- Propoxy, Methylthio, Ethylthio, n- oder i-Propylthio, Methylamino, Ethyl- amino, n- oder i-Propylamino, Dimethylamino, Diethylamino, Ethenyl, Propenyl, Butenyl, Ethinyl, Propinyl, Butinyl, Propenyloxy, Butenyloxy, Propinyloxy, Butinyloxy, Propenylthio, Butenylthio, Propinylthio, Butinyl- thio, oder für jeweils gegebenenfalls durch Fluor, Chlor oder Methyl sub- stituiertes Cyclopropyl, Cyclopropyloxy, Cyclopropylthio, Cyclopropyl- methyl, Cyclopropylmethoxy oder Cyclopropylmethylthio.
- R³: steht ganz besonders bevorzugt für Nitro, Cyano, Fluor, Chlor, Brom, für jeweils gegebenenfalls durch Fluor, Chlor, Methoxy oder Ethoxy sub- stituiertes Methyl, Ethyl, n- oder i-Propyl, Methoxy, Ethoxy, n- oder i- Propoxy, Methoxycarbonyl, Ethoxycarbonyl, n- oder i-Propoxycarbonyl, Methylthio, Ethylthio, n- oder i-Propylthio, Methylsulfinyl, Ethylsulfinyl, Methylsulfonyl oder Ethylsulfonyl, oder für jeweils gegebenenfalls durch Cyano, Nitro, Fluor, Chlor, Brom, Methyl, Ethyl, Trifluormethyl, Methoxy, Ethoxy, Difluormethoxy oder Trifluormethoxy substituiertes Phenyl oder Phenoxy.
- R⁴: steht ganz besonders bevorzugt für Wasserstoff, Nitro, Cyano, Fluor, Chlor, Brom, oder für jeweils gegebenenfalls durch Fluor, Chlor, Methoxy oder Ethoxy substituiertes substituiertes Methyl, Ethyl, n- oder i-Propyl, Methoxy, Ethoxy, n- oder i-Propoxy, Methoxycarbonyl, Ethoxycarbonyl, n- oder i- Propoxycarbonyl, Methylthio, Ethylthio, n- oder i-Propylthio, Methylsulfinyl, Ethylsulfinyl, Methylsulfonyl oder Ethylsulfonyl.

An Stelle der reinen Wirkstoffe der Formel (I) können auch Salze der Verbindungen der Formel (I) mit Metallen und/oder mit basischen Stickstoffverbindungen in den erfindungsgemäßen Wirkstoffkombinationen eingesetzt werden.

Hierbei werden Salze der Verbindungen der Formel (I) mit Alkalimetallen, wie z.B. Lithium, Natrium, Kalium, Rubidium oder Cäsium, ganz besonders mit Natrium oder Kalium, mit Erdalkalimetallen, wie z.B. Magnesium, Calcium oder Barium, ganz besonders mit Calcium, oder mit Erdmetallen wie z.B. Aluminium bevorzugt.

Weiter werden Salze der Verbindungen der Formel (I) mit Ammoniak, mit C₁-C₆-Alkyl-aminen, wie z.B. mit Methylamin, Ethylamin, n- oder i-Propylamin, n-, i-, s- oder t-Butylamin, n-, i-, s- oder t-Pentylamin, mit Di-(C₁-C₆-alkyl)-aminen, wie z.B. Dimethylamin, Diethylamin, Dipropylamin, Diisopropylamin, Dibutylamin, Diisobutylamin, Di-s-butylamin, Dipentylamin, Diisopentylamin, Di-s-pentylamin und Dihexylamin, mit Tri-(C₁-C₄-alkyl)-aminen, wie z.B. Trimethylamin, Triethylamin, Tripropylamin, Tributylamin und N-Ethyl-diisopropylamin, mit C₃-C₆-Cycloalkylaminen, wie z.B. Cyclopentylamin oder Cyclohexylamin, mit Di-(C₃-C₆-cycloalkyl)-aminen, wie z.B. Dicyclopentylamin oder Dicyclohexylamin, mit N-C₁-C₄-Alkyl-C₃-C₆-cycloalkylaminen, wie z.B. N-Methyl-cyclopentylamin, N-Ethyl-cyclopentylamin, N-Methyl-cyclohexylamin oder N-Ethyl-cyclohexylamin, mit N,N-Di-(C₁-C₄-alkyl)-C₃-C₆-cycloalkyl-aminen, wie z.B. N,N-Dimethyl-cyclopentylamin, N,N-Diethyl-cyclopentylamin, N,N-Dimethyl-cyclohexylamin oder N,N-Diethylcyclohexylamin, mit N-C₁-C₄-Alkyl-di-(C₃-C₆-cycloalkyl)-aminen, wie z.B. N-Methyl-dicyclopentylamin, N-Ethyl-dicyclopentylamin, N-Methyl-dicyclohexylamin oder N-Ethyl-dicyclohexylamin, mit Phenyl-C₁-C₄-alkyl-aminen, wie z.B. Benzylamin, 1-Phenyl-ethylamin oder 2-Phenyl-ethylamin, mit N-C₁-C₄-Alkyl-phenyl-C₁-C₄-alkyl-aminen, wie z.B. N-Methyl-benzylamin oder N-Ethylbenzylamin, oder mit N,N-Di-(C₁-C₄-alkyl)-phenyl-C₁-C₄-alkyl-aminen, wie z.B. N,N-Dimethyl-benzylamin oder N,N-Diethyl-benzylamin, oder mit gegebenenfalls annellierten und/oder durch C₁-C₄-Alkyl substituierten Azinen, wie z.B. Pyridin, Chinolin, 2-Methyl-pyridin, 3-Methyl-pyridin, 4-Methyl-pyridin, 2,4-Dimethyl-pyridin, 2,5-Dimethyl-pyridin, 2,6-Dimethyl-pyridin oder 5-Ethyl-2-methyl-pyridin bevorzugt.

Als basische Verbindungen, welche zur Herstellung der erfindungsgemäß einsetzbaren Salze der Verbindungen der Formel (I) verwendet werden können, seien genannt:
Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, n- oder i-propanolat, n-, i-, s- oder t-butanolat.

Als Beispiele für die als erfindungsgemäße Mischungspartner zu verwendenden Verbindungen der Formel (I) seien genannt:
2-(2-Chlor-phenylsulfonylaminocarbonyl)-, 2-(2-Brom-phenylsulfonylaminocarbonyl)-, 2-(2-Methyl-phenylsulfonylaminocarbonyl)-, 2-(2-Ethyl-phenylsulfonylaminocarbonyl)-, 2-(2-n-Propyl-phenylsulfonylaminocarbonyl)-, 2-(2-i-Propylphenylsulfonylaminocarbonyl)-, 2-(2-Trifluormethyl-phenylsulfonylaminocarbonyl)-, 2-(2-Methoxy-phenylsulfonylaminocarbonyl)-, 2-(2-Ethoxy-phenylsulfonylaminocarbo-nyl)-, 2-(2-n-Propoxy-phenylsulfonylaminocarbonyl)-, 2-(2-i-Propoxyphenylsulfonylaminocarbonyl)-, 2-(2-Difluormethoxy-phenylsulfonylaminocarbonyl)-, 2-(2-Trifluormethoxy-phenylsulfonylaminocarbonyl)-, 2-(2-Methoxycarbonyl-phenylsulfonylaminocarbonyl)-, 2-(2-Ethoxycarbonyl-phenylsulfonylaminocarbonyl)-, 2-(2-n-Propoxycarbonyl-phenylsulfonylaminocarbonyl)-, 2-(2-i-Propoxycarbonyl-phenylsulfonylaminocarbonyl)- und 2-(2-Chlor-6-methyl-phenylsulfonylaminocarbonyl)-4-methyl-5-methoxy-2,4-dihydro-3H-1,2,4-triazol-3-on, -4-methyl-5-ethoxy-2,4-dihydro-3H-1,2,4-triazol-3-on, -4-methyl-5-n-propoxy-2,4-dihydro-3H-1,2,4-triazol-3-on, -4-methyl-5-i-propoxy-2,4-dihydro-3H-1,2,4-triazol-3-on, -4-methyl-5-trifluorethoxy-2,4-dihydro-3H-1,2,4-triazol-3-on, -4-methyl-5-methylthio-2,4-dihydro-3H-1,2,4-triazol-3-on, -4-methyl-5-ethylthio-2,4-dihydro-3H-1,2,4-triazol-3-on, -4-methoxy-5-methyl-2,4-dihydro-3H-1,2,4-triazol-3-on, -4-methoxy-5-ethyl-2,4-dihydro-3H-1,2,4-triazol-3-on, -4-methoxy-5-n-propyl-2,4-dihydro-3H-1,2,4-triazol-3-on, -4-cyclopropyl-5-methoxy-2,4-dihydro-3H-1,2,4-triazol-3-on, -4-cyclopropyl-5-ethoxy-2,4-dihydro-3H-1,2,4-triazol-3-on, -4-cyclopropyl-5-n-propoxy-2,4-dihydro-3H-1,2,4-triazol-3-on, -4-cyclopropyl-5-i-propoxy-2,4-dihydro-3H-1,2,4-triazol-3-on und -4-cyclopropyl-5-trifluorethoxy-2,4-dihydro-3H-1,2,4-triazol-3-on sowie die Natrium- und Kalium-salze dieser Verbindungen.

Die Verbindungen 2-(2-Methoxycarbonyl-phenylsulfonylaminocarbonyl)-4-methyl-5-n-propoxy-2,4-dihydro-3H-1,2,4-triazol-3-on (I-1, Propoxycarbazone) und 2-(2-Trifluormethoxy-phenylsulfonylaminocarbonyl)-4-methyl-5-methoxy-2,4-dihydro-3H-1,2,4-triazol-3-on (I-2, Flucarbazone) sowie ihre Natriumsalze - (I-1-Na-Salz, Propoxycabazone-sodium, und 1-2-Na-Salz, Flucarbazone-sodium) - seien als Mischungskomponenten der Formel (I) besonders hervorgehoben.

Die Verbindungen der Formel (I) sind in den oben angegebenen Patentanmeldungen bzw. Patentschriften beschrieben.

Bevorzugte Bedeutungen der oben in Zusammenhang mit der allgemeinen Formel (II) aufgeführten Gruppen werden im Folgenden definiert.
- m: steht bevorzugt für die Zahlen 0, 1, 2, 3 oder 4.
- n: steht bevorzugt für die Zahlen 0, 1, 2 oder 3.
- X: steht bevorzugt für Nitro, Cyano, Carboxy, Carbamoyl, Formyl, Sulfamoyl, Hydroxy, Amino, Halogen, für jeweils gegebenenfalls durch Cyano, Halogen, Methoxy, Ethoxy, n- oder i-Propoxy substituiertes Methyl, Ethyl, n- oder i- Propyl, n-, i-, s- oder t-Butyl, Methoxy, Ethoxy, n- oder i-Propoxy, n-, i-, s- oder t-Butoxy, Methoxycarbonyl, Ethoxycarbonyl, n- oder i-Propoxy- carbonyl, n-, i-, s- oder t-Butoxycarbonyl, Methylthio, Ethylthio, n- oder i- Propylthio, n-, i-, s- oder t-Butylthio, Methylsulfinyl, Ethylsulfinyl, n- oder i- Propylsulfinyl, Methylsulfonyl, Ethylsulfonyl, Methylamino, Ethylamino, n- oder i-Propylamino, n-, i-, s- oder t-Butylamino, Methylaminocarbonyl, Ethylaminocarbonyl, n- oder i-Propylaminocarbonyl, oder für Dimethyl- amino, Diethylamino, Dimethylaminocarbonyl oder Dimethylaminosulfonyl.
- Y: steht bevorzugt für Nitro, Cyano, Carboxy, Carbamoyl, Formyl, Sulfamoyl, Hydroxy, Amino, Halogen, oder für jeweils gegebenenfalls durch Cyano, Halogen, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy.
- Z: steht bevorzugt für die nachstehende Gruppierung

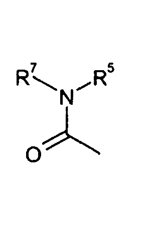

- worin R⁵: bevorzugt für Wasserstoff, für jeweils gegebenenfalls durch Cyano, Halogen, Methoxy, Ethoxy, n- oder i-Propoxy, n-, i-, s- oder t-Butoxy substituiertes Methyl, Ethyl, n- oder i-Propyl, n-, i-, s- oder t-Butyl, n-, i-, s- oder t-Pentyl, oder neo-Pentyl, für jeweils gegebenenfalls durch Halogen substituiertes Propenyl, Butenyl, Pentenyl, Propinyl, Butinyl oder Pentinyl, oder für jeweils gegebenenfalls durch Halogen, Methyl, Ethyl, n- oder i-Propyl substituiertes Cyclopropyl, Cyclobutyl, Cyclo- pentyl, Cyclohexyl, Cyclopropylmethyl, Cyclobutylmethyl, Cyclo- pentylmethyl oder Cyclohexylmethyl steht,
- R⁷: bevorzugt für Wasserstoff, für jeweils gegebenenfalls durch Cyano, Halogen, Methoxy, Ethoxy, n- oder i-Propoxy, n-, i-, s- oder t-Butoxy substituiertes Methyl, Ethyl, n- oder i-Propyl, n-, i-, s- oder t-Butyl, n-, i-, s- oder t-Pentyl, oder neo-Pentyl, für Methoxy, Ethoxy, n- oder i- Propoxy, n-, i-, s- oder t-Butoxy, für jeweils gegebenenfalls durch Halogen substituiertes Propenyl, Butenyl, Pentenyl, Propinyl, Butinyl oder Pentinyl, für Propenyloxy, Butenyloxy oder Pentenyloxy, für jeweils gegebenenfalls durch Halogen, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclopentylmethyl oder Cyclo- hexylmethyl, oder für jeweils gegebenenfalls durch Nitro, Cyano, Halogen, Methyl, Ethyl, n- oder i-Propyl, n-, i-, s- oder t-Butyl, Tri- fluormethyl, Methoxy, Ethoxy, n- oder i-Propoxy, Difluormethoxy oder Trifluormethoxy substituiertes Phenyl, Naphthyl, Benzyl oder Phenyl- ethyl steht, oder zusammen mit R⁵ für jeweils gegebenenfalls durch Methyl oder Ethyl substituiertes Butan-1,4-diyl (Tetramethylen), 1- Oxa-butan-1,4-diyl, Pentan-1,5-diyl, 1-Oxa-pentan-1,5-diyl oder 3-Oxa- pentan-1,5-diyl steht.

- m: steht besonders bevorzugt für die Zahlen 1 oder 2.
- n: steht besonders bevorzugt für die Zahl 0.
- X: steht besonders bevorzugt für Nitro, Cyano, Fluor, Chlor, Brom, für jeweils gegebenenfalls durch Fluor, Chlor, Methoxy oder Ethoxy substituiertes Methyl, Ethyl, n- oder i-Propyl, Methoxy, Ethoxy, n- oder i-Propoxy, Methoxycarbonyl, Ethoxycarbonyl, n- oder i-Propoxycarbonyl, Methylthio, Ethylthio, n- oder i-Propylthio, Methylsulfinyl, Ethylsulfinyl, Methylsulfonyl, Ethylsulfonyl, Methylamino, Ethylamino, n- oder i-Propylamino, Methyl- aminocarbonyl, Ethylaminocarbonyl, n- oder i-Propylaminocarbonyl, oder für Dimethylamino, Dimethylaminocarbonyl oder Dimethylaminosulfonyl.
- Z: steht besonders bevorzugt für die nachstehende Gruppierung

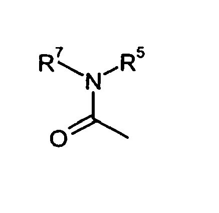

- worin R⁵: besonders bevorzugt für Wasserstoff, für gegebenenfalls durch Cyano, Fluor, Chlor, Methoxy, Ethoxy, n- oder i-Propoxy substituiertes Methyl, Ethyl, n- oder i-Propyl, n- oder i-Butyl, für jeweils gegebenen- falls durch Fluor und/oder Chlor substituiertes Propenyl, Butenyl, Propinyl oder Butinyl, oder für jeweils gegebenenfalls durch Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,

- R⁷: besonders bevorzugt für Wasserstoff, für jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, n-, i-, s- oder t- Pentyl, oder neo-Pentyl, für Methoxy, Ethoxy, n- oder i-Propoxy, n-, i-, s- oder t-Butoxy, für jeweils gegebenenfalls durch Fluor und/oder Chlor substituiertes Propenyl, Butenyl, Pentenyl, Propinyl, Butinyl oder Pentinyl, für Propenyloxy, Butenyloxy oder Pentenyloxy, für jeweils gegebenenfalls durch Fluor, Chlor, Methyl oder Ethyl substituiertes Cyclopropyl, Cyclobutyl, Cyclopentyl, Cyclohexyl, Cyclopropyl- methyl, Cyclobutylmethyl, Cyclopentylmethyl oder Cyclohexylmethyl, oder für jeweils gegebenenfalls durch Nitro, Cyano, Fluor, Chlor, Brom, Methyl, Ethyl, n- oder i-Propyl, n-, i-, s- oder t-Butyl, Trifluor- methyl, Methoxy, Ethoxy, n- oder i-Propoxy, Difluormethoxy oder Tri- fluormethoxy substituiertes Phenyl, Benzyl oder Phenylethyl steht, oder zusammen mit R⁵ für jeweils gegebenenfalls durch Methyl oder Ethyl substituiertes Butan-1,4-diyl (Tetramethylen), 1-Oxa-butan-1,4-diyl, Pentan-1,5-diyl, 1-Oxa-pentan-1,5-diyl oder 3-Oxa-pentan-1,5-diyl steht.

Eine Gruppe von erfindungsgemäß zu verwendenden Safenern sind die Verbindungen der allgemeinen Formel (IIb) 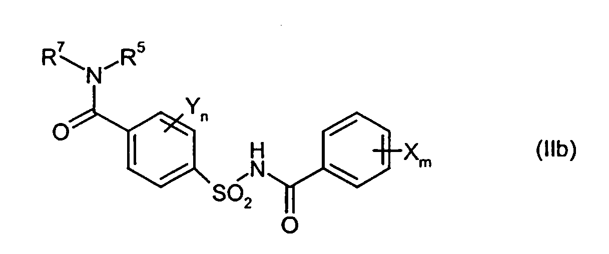 in welcher

m, n, R⁵, R⁷, X und Y die oben als bevorzugt bzw. als besonders bevorzugt angegebenen Bedeutungen haben.

Beispiele für die als erfindungsgemäß zu verwendenden Safener ganz besonders bevorzugten Verbindungen der Formel (IIb) sind in der nachstehenden Tabelle aufgeführt.

**Tabelle : Beispiele für die Verbindungen der Formel (IIb)**

| | | | | |
|---|---|---|---|---|
| 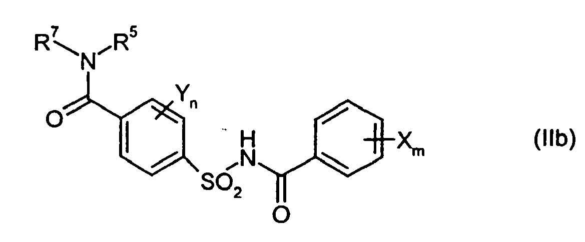 | | | | |

| **Bsp.-Nr.** | **R⁵** | **R⁷** | **(Position/en) Xₘ** | **(Position/en) Yₙ** |
|---|---|---|---|---|
| IIb-1 | H | H | (2) OCH₃ | - |
| IIb-2 | H | CH₃ | (2) OCH₃ | - |
| IIb-3 | H | CH₃ | (2) OCH₃ | - |
| IIb-4 | H | CH₃ | (2) OCH₃, (4) Cl | - |
| IIb-5 | H | CH₃ | (2) OCH₃, (4) CH₃ | - |
| IIb-6 | H | CH₃ | (2) OCH₃, (5) Cl | - |
| IIb-7 | H | CH₃ | (2) OCH₃, (4) Cl | - |
| IIb-8 | H | CH₃ | (2) OCH₃, (5) CH₃ | - |
| IIb-9 | H | CH₃ | (2) CF₃ | - |
| IIb-10 | H | CH₃ | (2) OCHF₂ | - |
| IIb-11 | H | CH₃ | (2) OCF₃ | - |
| IIb-12 | H | CH₃ | (2) Cl | - |
| IIb-13 | H | CH₃ | (2) OC₂H₅ | - |
| IIb-14 | H | C₂H₅ | (2) OCH₃ | - |
| IIb-15 | H | C₂H₅ | (2) OCH₃, (4) Cl | - |
| IIb-16 | H | C₂H₅ | (2) OCH₃, (4) CH₃ | - |
| IIb-17 | H | C₂H₅ | (2) OCH₃, (5) Cl | - |
| IIb-18 | H | C₂H₅ | (2) OCH₃, (4) Cl | - |
| IIb-19 | H | C₂H₅ | (2) OCH₃, (5) CH₃ | - |
| IIb-20 | H | C₂H₅ | (2) CF₃ | - |
| IIb-21 | H | C₂H₅ | (2) OCHF₂ | - |
| IIb-22 | H | C₂H₅ | (2) OCF₃ | - |
| IIb-23 | H | C₂H₅ | (2) Cl | - |
| IIb-24 | H | C₂H₅ | (2) OC₂H₅ | - |
| IIb-25 | H | C₂H₅ | (2) CH₃ | - |
| IIb-26 | H | C₂H₅ | (2) Cl, (4) Cl | - |
| IIb-27 | H | C₂H₅ | (2) Cl, (5) Cl | - |
| IIb-28 | H | C₂H₅ | (2) OCH₃, (5) OCH₃ - | |
| IIb-29 | H | C₂H₅ | (2) CH₃, (4) CH₃ | - |
| IIb-30 | H | C₂H₅ | (2) CH₃, (5) CH₃ | - |
| IIb-31 | H | C₃H₇-n | (2) OCH₃ | - |
| IIb-32 | H | C₃H₇-n | (2) OCH₃, (4) Cl | - |
| IIb-33 | H | C₃H₇-n | (2) OCH₃, (4) CH₃ | - |
| IIb-34 | H | C₃H₇-n | (2) OCH₃, (5) Cl | - |
| IIb-35 | H | C₃H₇-n | (2) OCH₃,(4) Cl | - |
| IIb-36 | H | C₃H₇-n | (2) OCH₃,(5) CH₃ | - |
| IIb-37 | H | C₃H₇-n | (2) CF₃ | - |
| IIb-38 | H | C₃H₇-n | (2) OCHF₂ | - |
| IIb-39 | H | C₃H₇-n | (2) OCF₃ | - |
| IIb-40 | H | C₃H₇-n | (2) Cl | - |
| IIb-41 | H | C₃H₇-n | (2) OC₂H₅ | - |
| IIb-42 | H | C₃H₇-n | (2) CH₃ | - |
| IIb-43 | H | C₃H₇-n | (2) Cl, (4) Cl | - |
| IIb-44 | H | C₃H₇-n | (2) Cl, (5) Cl | - |
| IIb-45 | H | C₃H₇-n | (2) OCH₃, (5) OCH₃ - | |
| IIb-46 | H | C₃H₇-n | (2) CH₃, (4) CH₃ | - |
| IIb-47 | H | C₃H₇-n | (2) CH₃, (5) CH₃ | - |
| IIb-48 | H | C₃H₇-i | (2) OCH₃ | - |
| IIb-49 | H | C₃H₇-i | (2) OCH₃, (4) Cl | - |
| IIb-50 | H | C₃H₇-i | (2) OCH₃, (4) CH₃ | - |
| IIb-51 | H | C₃H₇-i | (2) OCH₃, (5) Cl | - |
| IIb-52 | H | C₃H₇-i | (2) OCH₃, (4) Cl | - |
| IIb-53 | H | C₃H₇-i | (2) OCH₃, (5) CH₃ | - |
| IIb-54 | H | C₃H₇-i | (2) CF₃ | - |
| IIb-55 | H | C₃H₇-i | (2) OCHF₂ | - |
| IIb-56 | H | C₃H₇-i | (2) OCF₃ | - |
| IIb-57 | H | C₃H₇-i | (2) Cl | - |
| IIb-58 | H | C₃H₇-i | (2) OC₂H₅ | - |
| IIb-59 | H | C₃H₇-i | (2) CH₃ | - |
| IIb-60 | H | C₃H₇-i | (2) Cl, (4) Cl | - |
| IIb-61 | H | C₃H₇-i | (2) Cl, (5) Cl | - |
| IIb-62 | H | C₃H₇-i | (2) OCH₃, (5) OCH₃ - | |
| IIb-63 | H | C₃H₇-i | (2) CH₃, (4) CH₃ | - |
| IIb-64 | H | C₃H₇-i | (2) CH₃, (5) CH₃ | - |
| IIb-65 | H | CH₂CH=CH₂ | (2) OCH₃ | - |
| IIb-66 | H | CH₂CH=CH₂ | (2) OCH₃, (4) Cl | - |
| IIb-67 | H | CH₂CH=CH₂ | (2) OCH₃, (4) CH₃ | - |
| IIb-68 | H | CH₂CH=CH₂ | (2) OCH₃, (5) Cl | - |
| IIb-69 | H | CH₂CH=CH₂ | (2) OCH₃, (4) Cl | - |
| IIb-70 | H | CH₂CH=CH₂ | (2) OCH₃, (5) CH₃ | - |
| IIb-71 | H | CH₂CH=CH₂ | (2) CF₃ | - |
| IIb-72 | H | CH₂CH=CH₂ | (2) OCHF₂ | - |
| IIb-73 | H | CH₂CH=CH₂ | (2) OCF₃ | - |
| IIb-74 | H | CH₂CH=CH₂ | (2) Cl | - |
| IIb-75 | H | CH₂CH=CH₂ | (2) OC₂H₅ | - |
| IIb-76 | H | CH₂CH=CH₂ | (2) CH₃ | - |
| IIb-77 | H | CH₂CH=CH₂ | (2) Cl, (4) Cl | - |
| IIb-78 | H | CH₂CH=CH₂ | (2) Cl, (5) Cl | - |
| IIb-79 | H | CH₂CH=CH₂ | (2) OCH₃, (5) OCH₃ | - |
| IIb-80 | H | CH₂CH=CH₂ | (2) CH₃, (4) CH₃ | - |
| IIb-81 | H | CH₂CH=CH₂ | (2) CH₃, (5) CH₃ | - |
| IIb-82 | H | 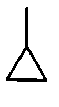 | (2) OCH₃ | - |
| IIb-83 | H | 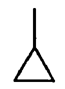 | (2) OCH₃, (4) Cl | - |
| IIb-84 | H | 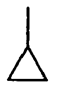 | (2) OCH₃, (4) CH₃ | - |
| IIb-85 | H | 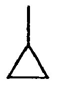 | (2) OCH₃, (5) Cl | - |
| IIb-86 | H | 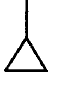 | (2) OCH₃, (4) Cl | - |
| IIb-87 | H | 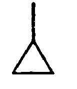 | (2) OCH₃, (5) CH₃ | - |
| IIb-88 | H | 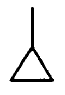 | (2) CF₃ | - |
| IIb-89 | H | 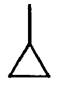 | (2) OCHF₂ | - |
| IIb-90 | H | 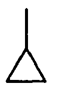 | (2) OCF₃ | - |
| IIb-91 | H | 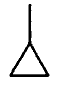 | (2) Cl | - |
| IIb-92 | H | 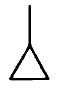 | (2) OC₂H₅ | - |
| IIb-93 | H | 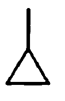 | (2) CH₃ | - |
| IIb-94 | H | 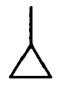 | (2) Cl, (4) Cl | - |
| IIb-95 | H | 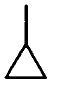 | (2) Cl, (5) Cl | - |
| IIb-96 | H | 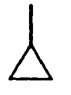 | (2) OCH₃, (5) OCH₃ - | |
| IIb-97 | H | 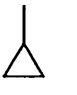 | (2) CH₃, (4) CH₃ | - |
| IIb-98 | H | 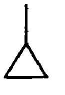 | (2) CH₃, (5) CH₃ | - |
| IIb-99 | H | C₄H₉-n | (2) OCH₃ | - |
| IIb-100 | H | C₄H₉-n | (2) OCH₃, (4) Cl | - |
| IIb-101 | H | C₄H₉-n | (2) OCH₃, (4) CH₃ | - |
| IIb-102 | H | C₄H₉-n | (2) OCH₃, (5) Cl | - |
| IIb-103 | H | C₄H₉-n | (2) OCH₃, (4) Cl | - |
| IIb-104 | H | C₄H₉-n | (2) OCH₃, (5) CH₃ | - |
| IIb-105 | H | C₄H₉-n | (2) CF₃ | - |
| IIb-106 | H | C₄H₉-n | (2) OCHF₂ | - |
| IIb-107 | H | C₄H₉-n | (2) OCF₃ | - |
| IIb-108 | H | C₄H₉-n | (2) Cl | - |
| IIb-109 | H | C₄H₉-n | (2) OC₂H₅ | - |
| IIb-110 | H | C₄H₉-n | (2) CH₃ | - |
| IIb-111 | H | C₄H₉-n | (2) Cl, (4) Cl | - |
| IIb-112 | H | C₄H₉-n | (2) Cl, (5) Cl | - |
| IIb-113 | H | C₄H₉-n | (2) OCH₃, (5) OCH₃ | - |
| IIb-114 | H | C₄H₉-n | (2) CH₃, (4) CH₃ | - |
| IIb-115 | H | C₄H₉-n | (2) CH₃, (5) CH₃ | - |
| IIb-116 | H | C₄H₉-i | (2) OCH₃ | - |
| IIb-117 | H | C₄H₉-i | (2) OCH₃, (4) Cl | - |
| IIb-118 | H | C₄H₉-i | (2) OCH₃, (4) CH₃ | - |
| IIb-119 | H | C₄H₉-i | (2) OCH₃, (5) Cl | - |
| IIb-120 | H | C₄H₉-i | (2) OCH₃, (4) Cl | - |
| IIb-121 | H | C₄H₉-i | (2) OCH₃, (5) CH₃ | - |
| IIb-122 | H | C₄H₉-i | (2) CF₃ | - |
| IIb-123 | H | C₄H₉-i | (2) OCHF₂ | - |
| IIb-124 | H | C₄H₉-i | (2) OCF₃ | - |
| IIb-125 | H | C₄H₉-i | (2) Cl | - |
| IIb-126 | H | C₄H₉-i | (2) OC₂H₅ | - |
| IIb-127 | H | C₄H₉-i | (2) CH₃ | - |
| IIb-128 | H | C₄H₉-i | (2) Cl, (4) Cl | - |
| IIb-129 | H | C₄H₉-i | (2) Cl, (5) Cl | - |
| IIb-130 | H | C₄H₉-i | (2) OCH₃, (5) OCH₃ - | |
| IIb-131 | H | C₄H₉-i | (2) CH₃, (4) CH₃ | - |
| IIb-132 | H | C₄H₉-i | (2) CH₃, (5) CH₃ | - |
| IIb-133 | H | C₄H₉-s | (2) OCH₃ | - |
| IIb-134 | H | C₄H₉-s | (2) OCH₃,(4) Cl | - |
| IIb-135 | H | C₄H₉-s | (2) OCH₃, (4) CH₃ | - |
| IIb-136 | H | C₄H₉-s | (2) OCH₃, (5) Cl | - |
| IIb-137 | H | C₄H₉-s | (2) OCH₃, (4) Cl | - |
| IIb-138 | H | C₄H₉-s | (2) OCH₃, (5) CH₃ | - |
| IIb-139 | H | C₄H₉-s | (2) CF₃ | - |
| IIb-140 | H | C₄H₉-s | (2) OCHF₂ | - |
| IIb-141 | H | C₄H₉-s | (2) OCF₃ | - |
| IIb-142 | H | C₄H₉-s | (2) Cl | - |
| IIb-143 | H | C₄H₉-s | (2) OC₂H₅ | - |
| IIb-144 | H | C₄H₉-s | (2) CH₃ | - |
| IIb-145 | H | C₄H₉-s | (2) Cl, (4) Cl | - |
| IIb-146 | H | C₄H₉-s | (2) Cl, (5) Cl | - |
| IIb-147 | H | C₄H₉-s | (2) OCH₃, (5) OCH₃ | - |
| IIb-148 | H | C₄H₉-s | (2) CH₃, (4) CH₃ | - |
| IIb-149 | H | C₄H₉-s | (2) CH₃, (5) CH₃ | - |
| IIb-150 | H | CH₂CH₂OCH₃ | (2) OCH₃ | - |
| IIb-151 | H | CH₂CH₂OCH₃ | (2) OCH₃, (4) Cl | - |
| IIb-152 | H | CH₂CH₂OCH₃ | (2) OCH₃, (4) CH₃ | - |
| IIb-153 | H | CH₂CH₂OCH₃ | (2) OCH₃, (5) Cl | - |
| IIb-154 | H | CH₂CH₂OCH₃ | (2) OCH₃, (4) Cl | - |
| IIb-155 | H | CH₂CH₂OCH₃ | (2) OCH₃, (5) CH₃ | - |
| IIb-156 | H | CH₂CH₂OCH₃ | (2) CF₃ | - |
| IIb-157 | H | CH₂CH₂OCH₃ | (2) OCHF₂ | - |
| IIb-158 | H | CH₂CH₂OCH₃ | (2) OCF₃ | - |
| IIb-159 | H | CH₂CH₂OCH₃ | (2) Cl | - |
| IIb-160 | H | CH₂CH₂OCH₃ | (2) OC₂H₅ | - |
| IIb-161 | H | CH₂CH₂OCH₃ | (2) CH₃ | - |
| IIb-162 | H | CH₂CH₂OCH₃ | (2) Cl, (4) Cl | - |
| IIb-163 | H | CH₂CH₂OCH₃ | (2) Cl, (5) Cl | - |
| IIb-164 | H | CH₂CH₂OCH₃ | (2) OCH₃, (5) OCH₃ - | |
| IIb-165 | H | CH₂CH₂OCH₃ | (2) CH₃, (4) CH₃ | - |
| IIb-166 | H | CH₂CH₂OCH₃ | (2) CH₃, (5) CH₃ | - |
| IIb-167 | H | 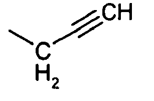 | (2) OCH₃ | - |
| IIb-168 | H | 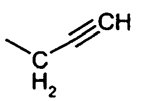 | (2) OCH₃, (4) Cl | - |
| IIb-169 | H | 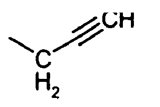 | (2) OCH₃, (4) CH₃ | - |
| IIb-170 | H | 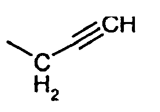 | (2) OCH₃, (5) Cl | - |
| IIb-171 | H | 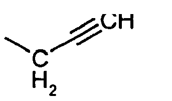 | (2) OCH₃, (4) Cl | - |
| IIb-172 | H | 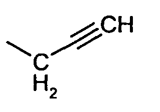 | (2) OCH₃, (5) CH₃ | - |
| IIb-173 | H | 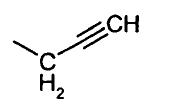 | (2) CF₃ | - |
| IIb-174 | H | 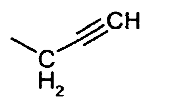 | (2) OCHF₂ | - |
| IIb-175 | H | 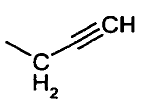 | (2) OCF₃ | - |
| IIb-176 | H | 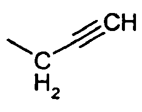 | (2) Cl | - |
| IIb-177 | H | 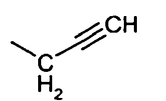 | (2) OC₂H₅ | - |
| IIb-178 | H | 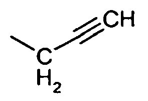 | (2) CH₃ | - |
| IIb-179 | H | 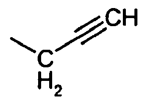 | (2) Cl, (4) Cl | - |
| IIb-180 | H | 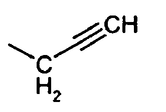 | (2) Cl, (5) Cl | - |
| IIb-181 | H | 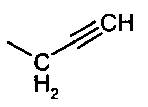 | (2) OCH₃, (5) OCH₃ - | |
| IIb-182 | H | 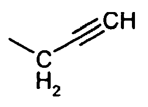 | (2) CH₃, (4) CH₃ | - |
| IIb-183 | H | 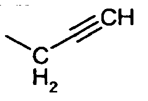 | (2) CH₃, (5) CH₃ | - |
| IIb-184 | H | CH₂CH₂OC₂H₅ | (2) OCH₃ | - |
| IIb-185 | H | CH₂CH₂OC₂H₅ | (2) OCH₃, (4) Cl | - |
| IIb-186 | H | CH₂CH₂OC₂H₅ | (2) OCH₃, (4) CH₃ | - |
| IIb-187 | H | CH₂CH₂OC₂H₅ | (2) OCH₃, (5) Cl | - |
| IIb-188 | H | CH₂CH₂OC₂H₅ | (2) OCH₃, (4) Cl | - |
| IIb-189 | H | CH₂CH₂OC₂H₅ | (2) OCH₃, (5) CH₃ | - |
| IIb-190 | H | CH₂CH₂OC₂H₅ | (2) CF₃ | - |
| IIb-191 | H | CH₂CH₂OC₂H₅ | (2) OCHF₂ | - |
| IIb-192 | H | CH₂CH₂OC₂H₅ | (2) OCF₃ | - |
| IIb-193 | H | CH₂CH₂OC₂H₅ | (2) Cl | - |
| IIb-194 | H | CH₂CH₂OC₂H₅ | (2) OC₂H₅ | - |
| IIb-195 | H | CH₂CH₂OC₂H₅ | (2) CH₃ | - |
| IIb-196 | H | CH₂CH₂OC₂H₅ | (2) Cl, (4) Cl | - |
| IIb-197 | H | CH₂CH₂OC₂H₅ | (2) Cl, (5) Cl | - |
| IIb-198 | H | CH₂CH₂OC₂H₅ | (2) OCH₃, (5) OCH₃ - | |
| IIb-199 | H | CH₂CH₂OC₂H₅ | (2) CH₃, (4) CH₃ | - |
| IIb-200 | H | CH₂CH₂OC₂H₅ | (2) CH₃, (5) CH₃ | - |
| IIb-201 | CH₃ | CH₃ | (2) OCH₃ | - |
| IIb-202 | CH₃ | CH₃ | (2) OCH₃, (4) Cl | - |
| IIb-203 | CH₃ | CH₃ | (2) OCH₃, (4) CH₃ | - |
| IIb-204 | CH₃ | CH₃ | (2) OCH₃, (5) Cl | - |
| IIb-205 | CH₃ | CH₃ | (2) OCH₃, (4) Cl | - |
| IIb-206 | CH₃ | CH₃ | (2) OCH₃, (5) CH₃ | - |
| IIb-207 | CH₃ | CH₃ | (2) CF₃ | - |
| IIb-208 | CH₃ | CH₃ | (2) OCHF₂ | - |
| IIb-209 | CH₃ | CH₃ | (2) OCF₃ | - |
| IIb-210 | CH₃ | CH₃ | (2) Cl | - |
| IIb-211 | CH₃ | CH₃ | (2) OC₂H₅ | - |
| IIb-212 | CH₃ | CH₃ | (2) CH₃ | - |
| IIb-213 | CH₃ | CH₃ | (2) Cl, (4) Cl | - |
| IIb-214 | CH₃ | CH₃ | (2) Cl, (5) Cl | - |
| IIb-215 | CH₃ | CH₃ | (2) OCH₃, (5) OCH₃ | - |
| IIb-216 | CH₃ | CH₃ | (2) CH₃, (4) CH₃ | - |
| IIb-217 | CH₃ | CH₃ | (2) CH₃, (5) CH₃ | - |
| IIb-218 | C₂H₅ | C₂H₅ | (2) OCH₃ | - |
| IIb-219 | C₂H₅ | C₂H₅ | (2) OCH₃, (4) Cl | - |
| IIb-220 | C₂H₅ | C₂H₅ | (2) OCH₃, (4) CH₃ | - |
| IIb-221 | C₂H₅ | C₂H₅ | (2) OCH₃, (5) Cl | - |
| IIb-222 | C₂H₅ | C₂H₅ | (2) OCH₃, (4) Cl | - |
| IIb-223 | C₂H₅ | C₂H₅ | (2) OCH₃, (5) CH₃ | - |
| IIb-224 | C₂H₅ | C₂H₅ | (2) CF₃ | - |
| IIb-225 | C₂H₅ | C₂H₅ | (2) OCHF₂ | - |
| IIb-226 | C₂H₅ | C₂H₅ | (2) OCF₃ | - |
| IIb-227 | C₂H₅ | C₂H₅ | (2) Cl | - |
| IIb-228 | C₂H₅ | C₂H₅ | (2) OC₂H₅ | - |
| IIb-229 | C₂H₅ | C₂H₅ | (2)CH₃ | - |
| IIb-230 | C₂H₅ | C₂H₅ | (2)Cl, (4)Cl | - |
| IIb-231 | C₂H₅ | C₂H₅ | (2)Cl, (5)Cl | - |
| IIb-232 | C₂H₅ | C₂H₅ | (2)OCH₃, (5)OCH₃ | - |
| IIb-233 | C₂H₅ | C₂H₅ | (2)CH₃, (4)CH₃ | - |
| IIb-234 | C₂H₅ | C₂H₅ | (2)CH₃, (5)CH₃ | |

Die Verbindungen der allgemeinen Formel (II) sind bekannt und/oder können nach an sich bekannten Verfahren hergestellt werden (vgl. WO-A-97/45016 / US-B1-6235680; WO-A-99/66795 / US-B1-6251827; WO-A-99/16744).

Als Beispiele für die erfindungsgemäßen selektiv herbiziden Kombinationen aus jeweils einem Wirkstoff der allgemeinen Formel (I) und jeweils einem Safener der allgemeinen Formel (II) seien besonders hervorgehoben:
Wirkstoff (I-1) - Propoxycarbazone - in Kombination mit den in der Tabelle aufgeführten Safenern, insbesondere (IIb-14) bis (IIb-98), (IIb-150) bis (IIb-234).
Wirkstoff (I-1-Na-Salz) - Propoxycarbazone-sodium - in Kombination mit den in der Tabelle aufgeführten Safenern, insbesondere (IIb-14) bis (IIb-98), (IIb-150) bis (IIb-234).
Wirkstoff (I-2) - Flucarbazone - in Kombination mit den in der Tabelle aufgeführten Safenern, insbesondere (IIb-14) bis (IIb-98), (IIb-150) bis (IIb-234).
Wirkstoff (I-2-Na-Salz) - Flucarbazone-sodium - in Kombination mit den in der Tabelle aufgeführten Safenern, insbesondere (IIb-14) bis (IIb-98), (IIb-150) bis (IIb-234).

Es wurde nun überraschend gefunden, dass die oben definierten Wirkstoffkombinationen aus substituierten Arylsulfonylaminocarbonyl-triazolinonen der allgemeinen Formel (I) und Safenern (Antidots) der allgemeinen Formel (II) bei sehr guter Nutzpflanzen-Verträglichkeit eine besonders hohe herbizide Wirksamkeit aufweisen und in verschiedenen Kulturen, insbesondere in Getreide (vor allem Gerste und Weizen), aber auch in Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Formel (II) geeignet sind, die schädigende Wirkung von substituierten Arylsulfonylaminocarbonyl-triazolinonen auf die Kulturpflanzen annähernd vollständig aufzuheben, ohne dabei die herbizide Wirksamkeit gegenüber den Unkräutern zu beeinträchtigen.

Hervorgehoben sei hierbei die besonders vorteilhafte Wirkung der besonders und am meisten bevorzugten Kombinationspartner der allgemeinen Formel (II), insbesondere hinsichtlich der Schonung von Getreidepflanzen, wie z.B. Weizen, Gerste und Roggen, aber auch Mais und Reis, als Kulturpflanzen.

Die erfindungsgemäßen Wirkstoffkombinationen können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
ikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita, Helianthus.
Monokotyle Unkräutern der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffkombinationen ist jedoch keineswegs auf diese Gattungen beschränkt, sondern erstreckt sich in gleicher Weise auch auf andere Pflanzen. Die Kulturpflanzen sind dabei erfindungsgemäß alle Pflanzen und Pflanzensorten einschließlich transgener Pflanzen und Pflanzensorten, wobei an transgenen Pflanzen und Pflanzensorten auch synergistische Effekte auftreten können.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) oder seinen Salzen 0,001 bis 1000 Gewichtsteile, vorzugsweise 0,01 bis 100 Gewichtsteile und besonders bevorzugt 0,1 bis 10 Gewichtsteile einer der oben unter (b) genannten, die Kulturpflanzen-Verträglichkeit verbessernden Verbindungen (Antidots/Safener).

Die Wirkstoffe bzw. Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nicht-ionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent an Wirkstoffen einschließlich der safenden Wirkstoffe, vorzugsweise zwischen 0,5 und 90%.

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Die neuen Wirkstoffkombinationen können als solche oder in ihren Formulierungen weiterhin auch in Mischung mit anderen bekannten Herbiziden Verwendung finden, wobei wiederum Fertigformulierungen oder Tankmischungen möglich sind. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstruktur-Verbesserungsmitteln ist möglich. Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,005 und 5 kg pro ha, vorzugsweise zwischen 0,01 und 2 kg pro ha, besonders bevorzugt zwischen 0,05 und 1,0 kg pro ha.

Die erfindungsgemäßen Wirkstoffkombinationen können vor und nach dem Auflaufen der Pflanzen appliziert werden, also im Vorauflauf und Nachauflauf-Verfahren.

### Anwendungsbeispiele

Die Wirkstoff (Flucarbazone-sodium und Propoxycarbazone-sodium wurden jeweils als WG 70 verwendet) bzw. -Safener-Komponenten werden jeweils in einigen ml (im Regelfall 2-3 ml) Lösungsmittel (im Regelfall Aceton oder N,N-Dimethylformamid) gelöst, die Lösungen vereinigt und dann - gegebenenfalls nach Zugabe eines Emulgators - mit Wasser auf die gewünschte Konzentration verdünnt. Es wurde im Regelfall eine wässrige Spritzbrühe mit 0,5 % des Additivs Renex-36 hergestellt.

### Beispiel A

### Post-emergence-Test

Die Testpflanzen werden unter kontrollierten Bedingungen (Temperatur, Licht, Luftfeuchte) im Gewächshaus aufgezogen. Die Spritzung wird durchgeführt, wenn die Testpflanzen eine Höhe von 5-15 cm erreicht haben. Die Konzentration der Spritzbrühe wird so gewählt, dass in 500 1 Wasser/ha die jeweils gewünschten Wirkstoffmengen ausgebracht werden.

Nach der Spritzung werden die Töpfe mit den Testpflanzen in einer Gewächshauskammer bis zum Testende unter kontrollierten Bedingungen (Temperatur, Licht, Luftfeuchte) gehalten. Etwa drei Wochen nach der Applikation wikrd der Schädigungsgrad der Kulturpflanzen bonitiert in % Schädigung im Vergleich zur Entwicklung der unbehandelten Kontrolle.

## Patentansprüche

1. Mittel, **gekennzeichnet durch** einen wirksamen Gehalt an einer Wirkstoffkombination umfassend
(a) ein oder mehrere substituierte Arylsulfonylaminocarbonyl-triazolinone der Formel (I) 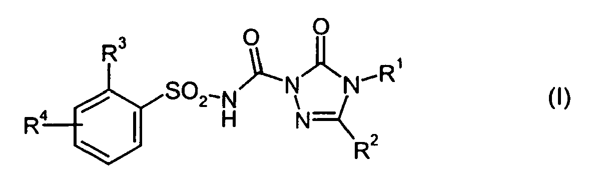 in welcher
R¹ für Wasserstoff, Hydroxy, Amino, C₂-C₆-Alkylidenamino, für jeweils gegebenenfalls **durch** Cyano, Halogen oder C₁-C₄-Alkoxy substitu- iertes Alkyl, Alkenyl, Alkinyl, Alkoxy, Alkenyloxy, Alkylamino oder Dialkylamino mit jeweils bis zu 6 Kohlenstoffatomen, für jeweils ge- gebenenfalls **durch** Cyano, Halogen oder C₁-C₄-Alkyl substituiertes Cycloalkyl, Cycloalkylalkyl oder Cycloalkylamino mit jeweils 3 bis 6 Kohlenstoffatomen in den Cycloalkylgruppen und gegebenenfalls 1 bis 4 Kohlenstoffatomen im Alkylteil, oder für jeweils gegebenenfalls **durch** Cyano, Nitro, Halogen, C₁-C₄-Alkyl, C₁-C₄-Halogenalkyl, C₁- C₄-Alkoxy oder C₁-C₄-Halogenalkoxy substituiertes Phenyl oder Phenyl-C₁-C₄-alkyl steht,
R² für Wasserstoff, Hydroxy, Mercapto, Amino, Cyano, Halogen, für jeweils gegebenenfalls **durch** Cyano, Halogen oder C₁-C₄-Alkoxy substituiertes Alkyl, Alkoxy, Alkylthio, Alkylamino, Dialkylamino, Alkenyl, Alkinyl, Alkenyloxy, Alkinyloxy, Alkenylthio, Alkinylthio, Alkenylamino oder Alkenylamino mit jeweils bis zu 6 Kohlenstoff- atomen, für jeweils gegebenenfalls **durch** Cyano, Halogen oder C₁- C₄-Alkyl substituiertes Cycloalkyl, Cycloalkyloxy, Cycloalkylthio, Cycloalkylamino, Cycloalkylalkyl, Cycloalkylalkoxy, Cycloalkyl- alkylthio oder Cycloalkylalkylamino mit jeweils 3 bis 6 Kohlenstoff- atomen in den Cycloalkylgruppen und gegebenenfalls 1 bis 4 Kohlen- stoffatomen im Alkylteil, oder für jeweils gegebenenfalls **durch** Cyano, Nitro, Halogen, C₁-C₄-Alkyl, C₁-C₄-Halogenalkyl, C₁-C₄- Alkoxy oder C₁-C₄-Halogenalkoxy substituiertes Phenyl, Phenoxy, Phenylthio, Phenylamino oder Phenyl-C₁-C₄-alkyl steht,
R³ für Nitro, Cyano, Halogen, für jeweils gegebenenfalls **durch** Cyano, Halogen oder C₁-C₄-Alkoxy substituiertes Alkyl, Alkylcarbonyl, Alkoxy, Alkoxycarbonyl, Alkylthio, Alkylsulfinyl, Alkylsulfonyl, Alkylamino, Alkenyl, Alkenyloxy, Alkenylthio, Alkenylamino, Alkinyl, Alkinyloxy, Alkinylthio mit jeweils bis zu 6 Kohlenstoff- atomen, für jeweils gegebenenfalls **durch** Cyano, Halogen oder C₁- C₄-Alkyl substituiertes Cycloalkyl, Cycloalkyloxy, Cycloalkylthio, Cycloalkylamino mit jeweils 3 bis 6 Kohlenstoffatomen in den Cyclo- alkylgruppen, oder für jeweils gegebenenfalls **durch** Cyano, Nitro, Halogen, C₁-C₄-Alkyl, C₁-C₄-Halogenalkyl, C₁-C₄-Alkoxy oder C₁- C₄-Halogenalkoxy substituiertes Phenyl, Phenoxy, Phenylthio, Phenylsulfinyl, Phenylsulfonyl oder Phenylamino steht, und
R⁴ für Wasserstoff, Nitro, Cyano, Halogen, für jeweils gegebenenfalls **durch** Cyano, Halogen oder C₁-C₄-Alkoxy substituiertes Alkyl, Alkylcarbonyl, Alkoxy, Alkoxycarbonyl, Alkylthio, Alkylsulfinyl, Alkylsulfonyl, Alkylamino, Alkenyl, Alkenyloxy, Alkenylthio, Alkenylamino, Alkinyl, Alkinyloxy oder Alkinylthio mit jeweils bis zu 6 Kohlenstoffatomen, für jeweils gegebenenfalls **durch** Cyano, Halogen oder C₁-C₄-Alkyl substituiertes Cycloalkyl, Cycloalkyloxy, Cycloalkylthio, Cycloalkylamino mit jeweils 3 bis 6 Kohlenstoff- atomen in den Cycloalkylgruppen, oder für jeweils gegebenenfalls **durch** Cyano, Nitro, Halogen, C₁-C₄-Alkyl, C₁-C₄-Halogenalkyl, C₁- C₄-Alkoxy oder C₁-C₄-Halogenalkoxy substituiertes Phenyl, Phenoxy, Phenylthio, Phenylsulfinyl, Phenylsulfonyl oder Phenyl- amino steht,
- einschließlich aller möglichen tautomeren Formen der Verbindungen der allgemeinen Formel (I) sowie der aus Verbindungen der allgemeinen Formel (I) und basischen Verbindungen gebildeten Salze -
("wirksame Verbindungen der Gruppe 1")
und
(b) zumindest eine die Kulturpflanzen-Verträglichkeit verbessernde Verbindung aus der folgenden Gruppe von Verbindungen der allgemeinen Formel (II) 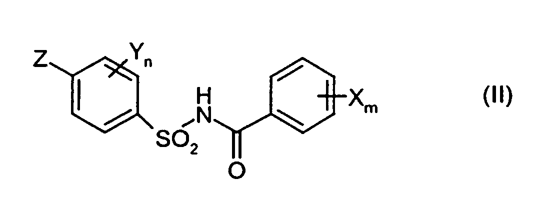 in welcher
m für die Zahlen 0 bis 5 steht,
n für die Zahlen 0 bis 4 steht,
X für Nitro, Cyano, Carboxy, Carbamoyl, Formyl, Sulfamoyl, Hydroxy, Amino, Halogen, oder für jeweils gegebenenfalls **durch** Cyano, Halogen oder C₁-C₄-Akoxy substituiertes Alkyl, Alkoxy, Alkoxycarbonyl, Alkylthio, Alkylsulfinyl, Alkyl- sulfonyl, Alkylamino, Alkylaminocarbonyl mit jeweils 1 bis 6 Kohlenstoffatomen in den Alkylgruppen, oder für Dialkyl- amino, Dialkylaminocarbonyl oder Dialkylaminosulfonyl mit jeweils 1 bis 6 Kohlenstoffatomen in den Alkylgruppen steht,
Y für Nitro, Cyano, Carboxy, Carbamoyl, Formyl, Sulfamoyl, Hydroxy, Amino, Halogen, oder für jeweils gegebenenfalls **durch** Cyano, Halogen oder C₁-C₄-Alkoxy substituiertes Alkyl oder Alkoxy mit jeweils 1 bis 6 Kohlenstoffatomen steht, und
Z für die nachstehende Gruppierung steht 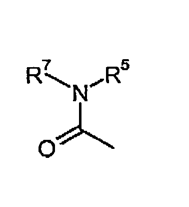
worin R⁵ für Wasserstoff, für gegebenenfalls **durch** Cyano, Halogen oder C₁-C₄-Alkoxy substituiertes Alkyl mit 1 bis 6 Kohlenstoffatomen, für jeweils gegebenenfalls **durch** Halogen substituiertes Alkenyl oder Alkinyl mit jeweils 3 bis 6 Kohlenstoffatomen, oder für jeweils ge- gebenenfalls **durch** Halogen oder C₁-C₄-Alkyl substitu- iertes Cycloalkyl oder Cycloalkylalkyl mit jeweils 3 bis 6 Kohlenstoffatomen in der Cycloalkylgruppe und ge- gebenenfalls 1 bis 4 Kohlenstoffatomen im Alkylteil steht,
R⁷ für Wasserstoff, für gegebenenfalls **durch** Cyano, Halogen oder C₁-C₄-Alkoxy substituiertes Alkyl mit 1 bis 6 Kohlenstoffatomen, für Alkoxy mit 1 bis 6 Kohlenstoffatomen, für jeweils gegebenenfalls **durch** Halogen substituiertes Alkenyl oder Alkinyl mit jeweils 3 bis 6 Kohlenstoffatomen, für Alkenyloxy mit 3 bis 6 Kohlenstoffatomen, für jeweils gegebenenfalls **durch** Halogen oder C₁-C₄-Alkyl substituiertes Cycloalkyl oder Cycloalkylalkyl mit 3 bis 6 Kohlenstoffatomen in der Cycloalkylgruppe und gegebenenfalls 1 bis 4 Kohlenstoffatomen im Alkylteil, oder für jeweils gege- benenfalls **durch** Nitro, Cyano, Halogen, C₁-C₄-Alkyl, C₁-C₄-Halohenalkyl, C₁-C₄-Alkoxy oder C₁-C₄- Halogenalkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil steht, oder zusammen mit R⁵ für jeweils gege- benenfalls **durch** C₁-C₄-Alkyl substituiertes Alkandiyl mit 2 bis 6 Kohlenstoffatomen oder Oxaalkandiyl mit 2 bis 5 Kohlenstoffatomen steht
("wirksame Verbindungen der Gruppe 2").

2. Mittel gemäß Anspruch 1, **dadurch gekennzeichnet, dass**
R¹ für Wasserstoff, Amino, Propylidenamino, Butylidenamino, für jeweils gegebenenfalls durch Cyano, Fluor, Chlor, Methoxy oder Ethoxy substituiertes Methyl, Ethyl, n- oder 1-Propyl, n-, i-, s- oder t- Butyl, Ethenyl, Propenyl, Butenyl, Ethinyl, Propinyl, Butinyl, Methoxy, Ethoxy, n- oder i-Propoxy, n-, i-, s- oder t-Butoxy, Propenyloxy, Butenyloxy, Methylamino, Ethylamino, n- oder i- Propylamino, n-, i-, s- oder t-Butylamino, Dimethylamino oder Di- ethylamino, für jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropylmethyl, Cyclo- butylmethyl, Cyclopentylmethyl, Cyclohexylmethyl, Cyclopropyl- amino, Cyclobutylamino, Cyclopentylamino oder Cyclohexylamino, oder für jeweils gegebenenfalls durch Cyano, Nitro, Fluor, Chlor, Brom, Methyl, Ethyl, n- oder i-Propyl, n-, i-, s- oder t-Butyl, Trifluor- methyl, Methoxy, Ethoxy, Difluormethoxy oder Trifluormethoxy sub- stituiertes Phenyl oder Benzyl steht,
R² für Wasserstoff, Hydroxy, Mercapto, Amino, Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t- Butoxy, Methylthio, Ethylthio, n- oder i-Propylthio, n-, i-, s- oder t- Butylthio, Methylamino, Ethylamino, n- oder i-Propylamino, n-, i-, s- oder t-Butylamino, Dimethylamino, Diethylamino, Ethenyl, Propenyl, Butenyl, Ethinyl, Propinyl, Butinyl, Propenyloxy, Butenyloxy, Propinyloxy, Butinyloxy, Propenylthio, Butenylthio, Propinylthio, Butinylthio, Propenylamino, Butenylamino, Propinylamino oder Butinylamino, für jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyl- oxy, Cyclopentyloxy, Cyclohexyloxy, Cyclopropylthio, Cyclobutyl- thio, Cyclopentylthio, Cyclohexylthio, Cyclopropylamino, Cyclo- butylamino, Cyclopentylamino, Cyclohexylamino, Cyclopropyl- methyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethyl- amino, Cyclobutylmethylamino, Cyclopentylmethylamino oder Cyclohexylmethylamino, oder für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, Difluormethoxy oder Trifluormethoxy substituiertes Phenyl, Phenoxy, Phenylthio, Phenylamino oder Benzyl steht,
R³ für Nitro, Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Acetyl, Propionyl, n- oder i-Butyroyl, Methoxy, Ethoxy, n- oder i-Propoxy, n-, i-, s- oder t- Butoxy, Methoxycarbonyl, Ethoxycarbonyl, n- oder i-Propoxy- carbonyl, Methylthio, Ethylthio, n- oder i-Propylthio, n-, i-, s- oder t- Butylthio, Methylsulfinyl, Ethylsulfinyl, n- oder i-Propylsulfinyl, Methylsulfonyl, Ethylsulfonyl, n- oder i-Propylsulfonyl, Methyl- amino, Ethylamino, n- oder i-Propylamino, n-, i-, s- oder t-Butyl- amino, Ethenyl, Propenyl, Butenyl, Propenyloxy, Butenyloxy, Propenylthio, Butenylthio, Propenylamino, Butenylamino, Ethinyl, Propinyl, Butinyl, Propinyloxy, Butinyloxy, Propinylthio oder Butinylthio, für jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyl- oxy, Cyclopentyloxy, Cyclohexyloxy, Cyclopropylthio, Cyclobutyl- thio, Cyclopentylthio, Cyclohexylthio, Cyclopropylamino, Cyclo- butylamino, Cyclopentylamino oder Cyclohexylamino, oder für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluor- methoxy oder Trifluormethoxy substituiertes Phenyl, Phenoxy, Phenylthio, Phenylsulfinyl, Phenylsulfonyl oder Phenylamino steht, und
R⁴ für Wasserstoff, Nitro, Cyano, Fluor, Chlor, Brom, für jeweils gege- benenfalls durch Cyano, Fluor, Chlor, Methoxy oder Ethoxy sub- stituiertes substituiertes Methyl, Ethyl, n- oder i-Propyl, n-, i-, s- oder t-Butyl, Acetyl, Propionyl, n- oder i-Butyroyl, Methoxy, Ethoxy, n- oder i-Propoxy, n-, i-, s- oder t-Butoxy, Methoxycarbonyl, Ethoxy- carbonyl, n- oder i-Propoxycarbonyl, Methylthio, Ethylthio, n- oder i- Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propyl- sulfonyl, Methylamino, Ethylamino, n- oder i-Propylamino, n-, i-, s- oder t-Butylamino, Ethenyl, Propenyl, Butenyl, Propenyloxy, Butenyl- oxy, Propenylthio, Butenylthio, Propenylamino, Butenylamino, Ethinyl, Propinyl, Butinyl, Propinyloxy, Butinyloxy, Propinylthio oder Butinylthio, für jeweils gegebenenfalls durch Cyano, Fluor, Chlor, Brom, Methyl, Ethyl, n- oder i-Propyl substituiertes Cyclo- propyl, Cyclobutyl, Cyclopentyl, Cyclohexyl, Cyclopropyloxy, Cyclo- butyloxy, Cyclopentyloxy, Cyclohexyloxy, Cyclopropylthio, Cyclo- butylthio, Cyclopentylthio, Cyclohexylthio, Cyclopropylamino, Cyclobutylamino, Cyclopentylamino oder Cyclohexylamino, oder für jeweils gegebenenfalls durch Cyano, Nitro, Fluor, Chlor, Brom, Methyl, Ethyl, n- oder i-Propyl, Trifluormethyl, Methoxy, Ethoxy, n- oder i-Propoxy, n-, i-, s- oder t-Butoxy, Difluormethoxy oder Trifluor- methoxy substituiertes Phenyl, Phenoxy, Phenylthio, Phenylsulfinyl, Phenylsulfonyl oder Phenylamino steht.

3. Mittel gemäß Anspruch 1, **dadurch gekennzeichnet, dass**
R¹ für Wasserstoff, Amino, für jeweils gegebenenfalls durch Fluor, Chlor, Methoxy oder Ethoxy substituiertes Methyl, Ethyl, Ethenyl, Propenyl, Ethinyl, Propinyl, Methoxy, Ethoxy, n- oder i-Propoxy, Methylamino oder Ethylamino, für Dimethylamino, oder für gege- benenfalls durch Fluor, Chlor oder Methyl substituiertes Cyclopropyl steht,
R² für Wasserstoff, Chlor, Brom, für jeweils gegebenenfalls durch Fluor, Chlor, Methoxy oder Ethoxy substituiertes Methyl, Ethyl, n- oder i- Propyl, n-, i- oder s-Butyl, Methoxy, Ethoxy, n- oder i-Propoxy, Methylthio, Ethylthio, n- oder i-Propylthio, Methylamino, Ethyl- amino, n- oder i-Propylamino, Dimethylamino, Diethylamino, Ethenyl, Propenyl, Butenyl, Ethinyl, Propinyl, Butinyl, Propenyloxy, Butenyloxy, Propinyloxy, Butinyloxy, Propenylthio, Butenylthio, Propinylthio, Butinylthio, Propenylamino, Butenylamino, Propinyl- amino oder Butinylamino, oder für jeweils gegebenenfalls durch Fluor, Chlor oder Methyl substituiertes Cyclopropyl, Cyclobutyl, Cyclopentyl, Cyclohexyl, Cyclopropyloxy, Cyclobutyloxy, Cyclo- pentyloxy, Cyclohexyloxy, Cyclopropylthio, Cyclobutylthio, Cyclo- pentylthio, Cyclohexylthio, Cyclopropylamino, Cyclobutylamino, Cyclopentylamino, Cyclohexylamino, Cyclopropylmethyl, Cyclo- butylmethyl, Cyclopentylmethyl, Cyclohexylmethyl, Cyclopropyl- methoxy, Cyclobutylmethoxy, Cyclopentylmethoxy, Cyclohexyl- methoxy, Cyclopropylmethylthio, Cyclobutylmethylthio, Cyclopentyl- methylthio, Cyclohexylmethylthio, Cyclopropylmethylamino, Cyclo- butylmethylamino, Cyclopentylmethylamino oder Cyclohexylmethyl- amino steht,
R³ für Nitro, Cyano, Fluor, Chlor, Brom, für jeweils gegebenenfalls durch Fluor, Chlor, Methoxy oder Ethoxy substituiertes Methyl, Ethyl, n- oder i-Propyl, Methoxy, Ethoxy, n- oder i-Propoxy, Methoxycarbonyl, Ethoxycarbonyl, n- oder i-Propoxycarbonyl, Methylthio, Ethylthio, n- oder i-Propylthio, Methylsulfinyl, Ethylsulfinyl, Methylsulfonyl oder Ethylsulfonyl, für jeweils gegebenenfalls durch Fluor, Chlor oder Methyl substituiertes Cyclopropyl, Cyclobutyl, Cyclopentyl, Cyclo- hexyl, Cyclopropyloxy, Cyclobutyloxy, Cyclopentyloxy, Cyclohexyl- oxy, Cyclopropylthio, Cyclobutylthio, Cyclopentylthio oder Cyclo- hexylthio, oder für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Phenylthio, Phenylsulfinyl oder Phenylsulfonyl steht, und
R⁴ für Wasserstoff, Nitro, Cyano, Fluor, Chlor, Brom, oder für jeweils gegebenenfalls durch Fluor, Chlor, Methoxy oder Ethoxy sub- stituiertes Methyl, Ethyl, n- oder i-Propyl, Methoxy, Ethoxy, n- oder i- Propoxy, Methoxycarbonyl, Ethoxycarbonyl, n- oder i-Propoxy- carbonyl, Methylthio, Ethylthio, n- oder i-Propylthio, Methylsulfinyl, Ethylsulfinyl, Methylsulfonyl oder Ethylsulfonyl steht.

4. Mittel gemäß Anspruch 1, **dadurch gekennzeichnet, dass**
R¹ für Wasserstoff, für jeweils gegebenenfalls durch Fluor, Chlor, Methoxy oder Ethoxy substituiertes Methyl, Ethyl, Ethenyl, Methoxy, Ethoxy, Methylamino oder Ethylamino, für Dimethylamino, oder für gegebenenfalls durch Fluor, Chlor oder Methyl substituiertes Cyclo- propyl steht,
R² für Wasserstoff, Chlor, Brom, für jeweils gegebenenfalls durch Fluor, Chlor, Methoxy oder Ethoxy substituiertes Methyl, Ethyl, n- oder i- Propyl, n-, i- oder s-Butyl, Methoxy, Ethoxy, n- oder i-Propoxy, Methylthio, Ethylthio, n- oder i-Propylthio, Methylamino, Ethyl- amino, n- oder i-Propylamino, Dimethylamino, Diethylamino, Ethenyl, Propenyl, Butenyl, Ethinyl, Propinyl, Butinyl, Propenyloxy, Butenyloxy, Propinyloxy, Butinyloxy, Propenylthio, Butenylthio, Propinylthio, Butinylthio, oder für jeweils gegebenenfalls durch Fluor, Chlor oder Methyl substituiertes Cyclopropyl, Cyclopropyloxy, Cyclopropylthio, Cyclopropylmethyl, Cyclopropylmethoxy oder Cyclopropylmethylthio steht,
R³ für Nitro, Cyano, Fluor, Chlor, Brom, für jeweils gegebenenfalls durch Fluor, Chlor, Methoxy oder Ethoxy substituiertes Methyl, Ethyl, n- oder i-Propyl, Methoxy, Ethoxy, n- oder i-Propoxy, Methoxycarbonyl, Ethoxycarbonyl, n- oder i-Propoxycarbonyl, Methylthio, Ethylthio, n- oder i-Propylthio, Methylsulfinyl, Ethylsulfinyl, Methylsulfonyl oder Ethylsulfonyl, oder für jeweils gegebenenfalls durch Cyano, Nitro, Fluor, Chlor, Brom, Methyl, Ethyl, Trifluormethyl, Methoxy, Ethoxy, Difluormethoxy oder Trifluormethoxy substituiertes Phenyl oder Phenoxy steht, und '
R⁴ für Wasserstoff, Nitro, Cyano, Fluor, Chlor, Brom, oder für jeweils gegebenenfalls durch Fluor, Chlor, Methoxy oder Ethoxy sub- stituiertes Methyl, Ethyl, n- oder i-Propyl, Methoxy, Ethoxy, n- oder i-Propoxy, Methoxycarbonyl, Ethoxycarbonyl, n- oder i-Propoxy- carbonyl, Methylthio, Ethylthio, n- oder i-Propylthio, Methylsulfinyl, Ethylsulfinyl, Methylsulfonyl oder Ethylsulfonyl steht.

5. Mittel gemäß Anspruch 1, **dadurch gekennzeichnet, dass**
m für die Zahlen 0, 1, 2, 3 oder 4 steht,
n für die Zahlen 0, 1, 2 oder 3 steht,
X für Nitro, Cyano, Carboxy, Carbamoyl, Formyl, Sulfamoyl, Hydroxy, Amino, Halogen, für jeweils gegebenenfalls durch Cyano, Halogen, Methoxy, Ethoxy, n- oder i-Propoxy substituiertes Methyl, Ethyl, n- oder i-Propyl, n-, i-, s- oder t-Butyl, Methoxy, Ethoxy, n- oder i- Propoxy, n-, i-, s- oder t-Butoxy, Methoxycarbonyl, Ethoxycarbonyl, n- oder i-Propoxycarbonyl, n-, i-, s- oder t-Butoxycarbonyl, Methyl- thio, Ethylthio, n- oder i-Propylthio, n-, i-, s- oder t-Butylthio, Methyl- sulfinyl, Ethylsulfinyl, n- oder i-Propylsulfinyl, Methylsulfonyl, Ethylsulfonyl, Methylamino, Ethylamino, n- oder i-Propylamino, n-, i-, s- oder t-Butylamino, Methylaminocarbonyl, Ethylaminocarbonyl, n- oder i-Propylaminocarbonyl, oder für Dimethylamino, Diethyl- amino, Dimethylaminocarbonyl oder Dimethylaminosulfonyl steht,
Y für Nitro, Cyano, Carboxy, Carbamoyl, Formyl, Sulfamoyl, Hydroxy, Amino, Halogen, oder für jeweils gegebenenfalls durch Cyano, Halogen, Methoxy, Ethoxy, n- oder i-Propoxy substituiertes Methyl, Ethyl, n- oder i-Propyl, n-; i-, s- oder t-Butyl, Methoxy, Ethoxy, n- oder i-Propoxy, n-, i-, s- oder t-Butoxy steht, und
Z für die nachstehende Gruppierung 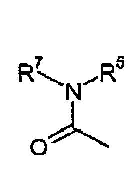 steht, worin
R⁵ für Wasserstoff, für jeweils gegebenenfalls durch Cyano, Halogen, Methoxy, Ethoxy, n- oder i-Propoxy, n-, i-, s- oder t- Butoxy substituiertes Methyl, Ethyl, n- oder i-Propyl, n-, i-, s- oder t-Butyl, n-, i-, s- oder t-Pentyl, oder neo-Pentyl, für jeweils gegebenenfalls durch Halogen substituiertes Propenyl, Butenyl, Pentenyl, Propinyl, Butinyl oder Pentinyl, oder für jeweils gegebenenfalls durch Halogen, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclo- pentylmethyl oder Cyclohexylmethyl steht,
R⁷ für Wasserstoff, für jeweils gegebenenfalls durch Cyano, Halogen, Methoxy, Ethoxy, n- oder i-Propoxy, n-, i-, s- oder t- Butoxy substituiertes Methyl, Ethyl, n- oder i-Propyl, n-, i-, s- oder t-Butyl, n-, i-, s- oder t-Pentyl, oder neo-Pentyl, für Methoxy, Ethoxy, n- oder i-Propoxy, n-, i-, s- oder t-Butoxy, für jeweils gegebenenfalls durch Halogen substituiertes Propenyl, Butenyl, Pentenyl, Propinyl, Butinyl oder Pentinyl, für Propenyloxy, Butenyloxy oder Pentenyloxy, für jeweils ge- gebenenfalls durch Halogen, Methyl, Ethyl, n- oder i-Propyl substituiertes Cyclopropyl, Cyclobutyl, Cyclopentyl, Cyclo- hexyl, Cyclopropylmethyl, Cyclobutylmethyl, Cyclopentyl- methyl oder Cyclohexylmethyl, oder für jeweils gegebenen- falls durch Nitro, Cyano, Halogen, Methyl, Ethyl, n- oder i- Propyl, n-, i-, s- oder t-Butyl, Trifluormethyl, Methoxy, Ethoxy, n- oder i-Propoxy, Difluormethoxy oder Trifluor- methoxy substituiertes Phenyl, Naphthyl, Benzyl oder Phenyl- ethyl steht, oder zusammen mit R⁵ für jeweils gegebenenfalls durch Methyl oder Ethyl substituiertes Butan-1,4-diyl (Tetra- methylen), 1-Oxa-butan-1,4-diyl, Pentan-1,5-diyl, 1-Oxa- pentan-1,5-diyl oder 3-Oxa-pentan-1,5-diyl steht.

6. Mittel gemäß Anspruch 1, **dadurch gekennzeichnet, dass**
m für die Zahlen 1 oder 2 steht,
n für die Zahl 0 steht,
X für Nitro, Cyano, Fluor, Chlor, Brom, für jeweils gegebenenfalls durch Fluor, Chlor, Methoxy oder Ethoxy substituiertes Methyl, Ethyl, n- oder i-Propyl, Methoxy, Ethoxy, n- oder 1-Propoxy, Methoxycarbonyl, Ethoxycarbonyl, n- oder i-Propoxycarbonyl, Methylthio, Ethylthio, n- oder i-Propylthio, Methylsulfinyl, Ethylsulfinyl, Methylsulfonyl, Ethylsulfonyl, Methylamino, Ethylamino, n- oder i-Propylamino, Methylaminocarbonyl, Ethylaminocarbonyl, n- oder i-Propylamino- carbonyl, oder für Dimethylamino, Dimethylaminocarbonyl oder Di- methylaminosulfonyl steht, und
Z für die nachstehende Gruppierung 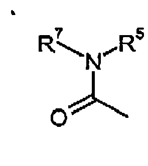 steht, worin
R⁵ für Wasserstoff, für jeweils gegebenenfalls durch Cyano, Fluor, Chlor, Methoxy, Ethoxy, n- oder i-Propoxy substitu- iertes Methyl, Ethyl, n- oder i-Propyl, n- oder i-Butyl, für jeweils gegebenenfalls durch Fluor und/oder Chlor substitu- iertes Propenyl, Butenyl, Propinyl oder Butinyl, oder für jeweils gegebenenfalls durch Fluor, Chlor, Methyl oder Ethyl substituiertes Cyclopropyl, Cyclobutyl, Cyclopentyl, Cyclo- hexyl, Cyclopropylmethyl, Cyclobutyhnethyl, Cyclopentyl- methyl oder Cyclohexylmethyl steht,
R⁷ für Wasserstoff, für jeweils gegebenenfalls durch Cyano, Fluor, Chlor, Methoxy, Ethoxy, n- oder i-Propoxy substitu- iertes Methyl, Ethyl, n- oder i-Propyl, n-, i-, s- oder t-Butyl, n-, i-, s- oder t-Pentyl, oder neo-Pentyl, für Methoxy, Ethoxy, n- oder i-Propoxy, n-, i-, s- oder t-Butoxy, für jeweils gegebenen- falls durch Fluor und/oder Chlor substituiertes Propenyl, Butenyl, Pentenyl, Propinyl, Butinyl oder Pentinyl, für Propenyloxy, Butenyloxy oder Pentenyloxy, für jeweils gege- benenfalls durch Fluor, Chlor, Methyl oder Ethyl substituiertes Cyclopropyl, Cyclobutyl, Cyclopentyl, Cyclohexyl, Cyclo- propylmethyl, Cyclobutylmethyl, Cyclopentylmethyl oder Cyclohexylmethyl, oder für jeweils gegebenenfalls durch Nitro, Cyano, Fluor, Chlor, Brom, Methyl, Ethyl, n- oder i- Propyl, n-, i-, s- oder t-Butyl, Trifluormethyl, Methoxy, Ethoxy, n- oder i-Propoxy, Difluormethoxy oder Trifluor- methoxy substituiertes Phenyl, Benzyl oder Phenylethyl steht, oder zusammen mit R⁵ für jeweils gegebenenfalls durch Methyl oder Ethyl substituiertes Butan-1,4-diyl (Tetra- methylen), 1-Oxa-butan-1,4-diyl, Pentan-1,5-diyl, 1-Oxa- pentan-1,5-diyl oder 3-Oxa-pentan-1,5-diyl steht.

7. Mittel gemäß einem der Ansprüche 1 bis 6, **dadurch gekennzeichnet dass**, die Verbindung der Formel (I) eine oder mehrere Verbindungen ausgewählt aus den Verbindungen 2-(2-Methoxycarbonyl-phenylsulfonylaminocarbonyl)-4-methyl-5-n-propoxy-2,4-dihydro-3H-1,2,4-triazol-3-on (Propoxycarbazone), 2-(2-Trifluormethoxy-phenylsulfonylaminocarbonyl)-4-methyl-5-methoxy-2,4-dihydro-3H-1,2,4-triazol-3-on (Flucarbazone) und den entsprechenden Natriumsalzen (Propoxycarbazone-sodium bzw. Flucarbazone-sodium) ist.

8. Verwendung eines Mittels gemäß einem der Ansprüche 1 bis 7 zur Bekämpfung von unerwünschten Pflanzen.

9. Verfahren zur Bekämpfung von unerwünschten Pflanzen, **dadurch gekennzeichnet, dass** man Mittel gemäß einem der Ansprüche 1 bis 7 auf die unerwünschten Pflanzen und/oder ihren Lebensraum einwirken lässt.

10. Verfahren zur Herstellung eines herbiziden Mittels, **dadurch gekennzeichnet, dass** man ein Mittel gemäß einem der Ansprüche 1 bis 7 mit oberflächenaktiven Mitteln und/oder Streckmitteln vermischt.

## Claims

1. Composition, **characterized by** an effective amount of a combination of active agents comprising
(a) one or more substituted arylsulphonylaminocarbonyl-triazolinones of Formula (I) 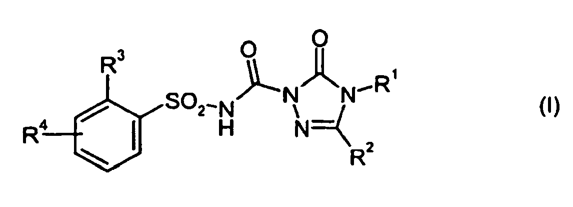 in which
R¹ stands for hydrogen, hydroxy, amino, C₂-C₆-alkylidenamino, for alkyl, alkenyl, alkinyl, alkoxy, alkenyloxy, alkylamino or dialkylamino with up to 6 carbon atoms each, in each instance optionally substituted with cyano, halogen or C₁-C₄-alkoxy, for cycloalkyl, cycloalkylalkyl or cycloalkylamino with 3 to 6 carbon atoms in the cycloalkyl groups and optionally 1 to 4 carbon atoms in the alkyl moiety each, in each instance optionally substituted with cyano, halogen or C₁-C₄-alkyl, or for phenyl or phenyl-C₁- C₄-alkyl, in each instance optionally substituted with cyano, nitro, halogen, C₁-C₄-alkyl, C₁-C₄-halogenalkyl, C₁-C₄-alkoxy or C₁-C₄-halogenalkoxy,
R² stands for hydrogen, hydroxy, mercapto, amino, cyano, halogen, for alkyl, alkoxy, alkylthio, alkylamino, dialkylamino, alkenyl, alkinyl, alkenyloxy, alkinyloxy, alkenylthio, alkinylthio, alkenylamino or alkinylamino with up to 6 carbon atoms each, in each instance optionally substituted with cyano, halogen or C₁-C₄-alkoxy, for cycloalkyl, cycloalkyloxy, cycloalkylthio, cycloalkylamino, cycloalkylalkyl, cycloalkylalkoxy, cycloalkylalkylthio or cycloalkylalkylamino with 3 to 6 carbon atoms in the cycloalkyl groups and optionally 1 to 4 carbon atoms in the alkyl moiety each, in each instance optionally substituted with cyano, halogen or C₁-C₄-alkyl, or for phenyl, phenoxy, phenylthio, phenylamino or phenyl-C₁-C₄-alkyl, in each instance optionally substituted with cyano, nitro, halogen, C₁-C₄-alkyl, C₁-C₄- halogenalkyl, C₁-C₄-alkoxy or C₁-C₄-halogenalkoxy,
R³ stands for nitro, cyano, halogen, for alkyl, alkylcarbonyl, alkoxy, alkoxycarbonyl, alkylthio, alkylsulphinyl, alkylsulphonyl, alkylamino, alkenyl, alkenyloxy, alkenylthio, alkenylamino, alkinyl, alkinyloxy, alkinylthio with up to 6 carbon atoms each, in each instance optionally substituted with cyano, halogen or C₁-C₄-alkoxy, for cycloalkyl, cycloalkyloxy, cycloalkylthio, cycloalkylamino with 3 to 6 carbon atoms in the cycloalkyl groups each, in each instance optionally substituted with cyano, halogen or C₁-C₄-alkyl, or for phenyl, phenoxy, phenylthio, phenylsulphinyl, phenylsulphonyl or phenylamino, in each instance optionally substituted with cyano, nitro, halogen, C₁-C₄-alkyl, C₁-C₄-halogenalkyl, C₁-C₄-alkoxy or C₁-C₄ halogenalkoxy, and
R⁴ stands for hydrogen, nitro, cyano, halogen, for alkyl, alkylcarbonyl, alkoxy, alkoxycarbonyl, alkylthio, alkylsulphinyl, alkylsulphonyl, alkylamino, alkenyl, alkenyloxy, alkenylthio, alkenylamino, alkinyl, alkinyloxy or alkinylthio with up to 6 carbon atoms each, in each instance optionally substituted with cyano, halogen or C₁-C₄-alkoxy, for cycloalkyl, cycloalkyloxy, cycloalkylthio, cycloalkylamino with 3 to 6 carbon atoms in the cycloalkyl groups each, in each instance optionally substituted with cyano, halogen or C₁-C₄-alkyl, or for phenyl, phenoxy, phenylthio, phenylsulphinyl, phenylsulphonyl or phenylamino, in each instance optionally substituted with cyano, nitro, halogen, C₁-C₄-alkyl, C₁-C₄-halogenalkyl, C₁-C₄-alkoxy or C₁-C₄ halogenalkoxy,
- including all possible tautomeric forms of compounds of the general Formula (I) as well as salts formed from compounds of general Formula (I) and basic compounds
("effective compounds of group 1")
and
(b) at least one compound improving compatibility with cultivated plants, said at least one compound being selected from the following group of compounds of the general Formula (II) 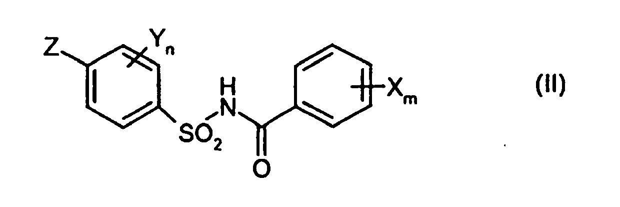 in which
m stands for the numbers 0 to 5,
n stands for the numbers 0 to 4,
X stands for nitro, cyano, carboxy, carbamoyl, formyl, sulfamoyl, hydroxy, amino, halogen or for alkyl, alkoxy, alkoxycarbonyl, alkylthio, alkylsulphinyl, alkylsulphonyl, alkylamino, alkylaminocarbonyl with 1 to 6 carbon atoms in the alkyl groups each, in each instance optionally substituted with cyano, halogen or C₁-C₄-alkoxy or for dialkylamino, dialkylaminocarbonyl or dialkylaminosulphonyl with 1 to 6 carbon atoms in the alkyl groups,
Y stands for nitro, cyano, carboxy, carbamoyl, formyl, sulfamoyl, hydroxy, amino, halogen or for alkyl or alkoxy with 1 to 6 carbon atoms each, in each instance optionally substituted with cyano, halogen or C₁-C₄-alkoxy, and
Z stands for the following group 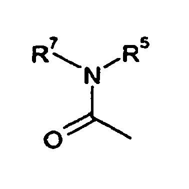 wherein
R⁵ stands for hydrogen, for alkyl with 1 to 6 carbon atoms, optionally substituted with cyano, halogen or C₁-C₄-alkoxy, for alkenyl or alkinyl with 3 to 6 carbon atoms each, in each instance optionally substituted with halogen, or for cycloalkyl or cycloalkylalkyl, each with 3 to 6 carbon atoms in the cycloalkyl group and optionally 1 to 4 carbon atoms in the alkyl moiety, in each instance optionally substituted with halogen or C₁-C₄-alkyl, and
R⁷ stands for hydrogen, alkyl with 1 to 6 carbon atoms, optionally substituted with cyano, halogen or C₁-C₄-alkoxy, for alkoxy with 1 to 6 carbon atoms, for alkenyl or alkinyl with 3 to 6 carbon atoms each, in each instance optionally substituted with halogen, for alkenyloxy with 3 to 6 carbon atoms, for cycloalkyl or cycloalkylalkyl with 3 to 6 carbon atoms in the cycloalkyl group and optionally 1 to 4 carbon atoms in the alkyl moiety, in each instance optionally substituted with halogen or C₁-C₄-alkyl, or for aryl or arylalkyl each with 6 or 10 carbon atoms in the aryl group and optionally 1 to 4 carbon atoms in the alkyl moiety, in each instance optionally substituted with nitro, cyano, halogen, C₁- C₄-alkyl, C₁-C₄-halogenalkyl, C₁-C₄-alkoxy or C₁-C₄-halogenalkoxy, or, together with R⁵, for alkandiyl with 2 to 6 carbon atoms or oxaalkandiyl with 2 to 5 carbon atoms, in each instance optionally substituted with C₁-C₄-alkyl
("effective compounds of group 2").

2. Composition according to claim 1, **characterized in that**
R¹ stands for hydrogen, amino, propylidenamino, butylidenamino, for methyl, ethyl, n- or i-propyl, n-, i-, s- or t-butyl, ethenyl, propenyl, butenyl, ethinyl, propinyl, butinyl, methoxy, ethoxy, n- or i-propoxy, n-, i-, s- or t-butoxy, propenyloxy, butenyloxy, methylamino, ethylamino, n- or i-propylamino, n-, i-, s- or t-butylamino, dimethylamino or diethylamino, in each instance optionally substituted with cyano, fluorine, chlorine, methoxy or ethoxy, for cyclopropyl, cyclobutyl, cyclopentyl, cyclohexyl, cyclopropylmethyl, cyclobutylmethyl, cyclopentylmethyl, cyclohexylmethyl, cyclopropylamino, cyclobutylamino, cyclopentylamino or cyclohexylamino, in each instance optionally substituted with cyano, fluorine, chlorine, bromine, methyl, ethyl, n- or i-propyl, or for phenyl or benzyl, in each instance optionally substituted with cyano, nitro, fluorine, chlorine, bromine, methyl, ethyl, n- or i-propyl, n-, i-, s- or t-butyl, trifluoromethyl, methoxy, ethoxy, difluoromethoxy or trifluoromethoxy,
R² stands for hydrogen, hydroxy, mercapto, amino, cyano, fluorine, chlorine, bromine, for methyl, ethyl, n- or i-propyl, n-, i-, s- or t-butyl, methoxy, ethoxy, n- or i-propoxy, n-, i-, s- or t-butoxy, methylthio, ethylthio, n- or i- propylthio, n-, i-, s- or t-butylthio, methylamino, ethylamino, n- or i- propylamino, n-, i-, s- or t-butylamino, dimethylamino, diethylamino, ethenyl, propenyl, butenyl, ethinyl, propinyl, butinyl, propenyloxy, butenyloxy, propinyloxy, butinyloxy, propenylthio, butenylthio, propinylthio, butinylthio, propenylamino, butenylamino, propinylamino or butinylamino, in each instance optionally substituted with cyano, fluorine, chlorine, methoxy or ethoxy, for cyclopropyl, cyclobutyl, cyclopentyl, cyclohexyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopropylmethylthio, cyclobutylmethylthio, cyclopentylmethylthio, cyclohexylmethylthio, cyclopropylmethylamino, cyclobutylmethylamino, cyclopentylmethylamino or cyclohexylmethylamino, in each instance optionally substituted with cyano, fluorine, chlorine, bromine, methyl, ethyl, n- or i-propyl, or for phenyl, phenoxy, phenylthio, phenylamino or benzyl, in each instance optionally substituted with cyano, nitro, fluorine, chlorine, bromine, methyl, ethyl, n- or i-propyl, n-, i-, s- or t- butyl, trifluoromethyl, methoxy, ethoxy, difluoromethoxy or trifluoromethoxy,
R³ stands for nitro, cyano, fluorine, chlorine, bromine, for methyl, ethyl, n- or i- propyl, n-, i-, s- or t-butyl, acetyl, propionyl, n- or i-butyroyl, methoxy, ethoxy, n- or i-propoxy, n-, i-, s- or t-butoxy, methoxycarbonyl, ethoxycarbonyl, n- or i-propoxycarbonyl, methylthio, ethylthio, n- or i- propylthio, n-, i-, s- or t-butylthio, methylsulphinyl, ethylsulphinyl, n- or i- propylsulphinyl, methylsulphonyl, ethylsulphonyl, n- or i-propylsulphonyl, methylamino, ethylamino, n- or i-propylamino, n-, i- s- or t-butylamino, ethenyl, propenyl, butenyl, propenyloxy, butenyloxy, propenylthio, butenylthio, propenylamino, butenylamino, ethinyl, propinyl, butinyl, propinyloxy, butinyloxy, propinylthio or butinylthio, in each instance optionally substituted with cyano, fluorine, chlorine, methoxy or ethoxy, for cyclopropyl, cyclobutyl, cyclopentyl, cyclohexyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino or cyclohexylamino, in each instance optionally substituted with cyano, fluorine, chlorine, bromine, methyl, ethyl, n- or i-propyl, or for phenyl, phenoxy, phenylthio, phenylsulphinyl, phenylsulphonyl or phenylamino, in each instance optionally substituted with cyano, nitro, fluorine, chlorine, bromine, methyl, ethyl, n- or i-propyl, n-, i-, s- or t-butyl, trifluoromethyl, methoxy, ethoxy, n- or i-propoxy, n-, i-, s- or t-butoxy, difluoromethoxy or trifluoromethoxy, and
R⁴ stands for hydrogen, nitro, cyano, fluorine, chlorine, bromine, for methyl, ethyl, n- or i-propyl, n-, i-, s- or t-butyl, acetyl, propionyl, n- or i-butyroyl, methoxy, ethoxy, n- or i-propoxy, n-, i-, s- or t-butoxy, methoxycarbonyl, ethoxycarbonyl, n- or i-propoxycarbonyl, methylthio, ethylthio, n- or i- propylthio, n-, i-, s- or t-butylthio, methylsulphinyl, ethylsulphinyl, n- or i- propylsulphinyl, methylsulphonyl, ethylsulphonyl, n- or i-propylsulphonyl, methylamino, ethylamino, n- or i-propylamino, n-, i- s- or t-butylamino, ethenyl, propenyl, butenyl, propenyloxy, butenyloxy, propenylthio, butenylthio, propenylamino, butenylamino, ethinyl, propinyl, butinyl, propinyloxy, butinyloxy, propinylthio or butinylthio, in each instance optionally substituted with cyano, fluorine, chlorine, methoxy or ethoxy, for cyclopropyl, cyclobutyl, cyclopentyl, cyclohexyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino or cyclohexylamino, in each instance optionally substituted with cyano, fluorine, chlorine, bromine, methyl, ethyl, n- or i-propyl, or for phenyl, phenoxy, phenylthio, phenylsulphinyl, phenylsulphonyl or phenylamino, in each instance optionally substituted with cyano, nitro, fluorine, chlorine, bromine, methyl, ethyl, n- or i-propyl, trifluoromethyl, methoxy, ethoxy, n- or i-propoxy, n-, i-, s- or t-butoxy, difluoromethoxy or trifluoromethoxy.

3. Composition according to claim 1, **characterized in that**
R¹ stands for hydrogen, amino, for methyl, ethyl, ethenyl, propenyl, ethinyl, propinyl, methoxy, ethoxy, n- or i-propoxy, methylamino or ethylamino, in each instance optionally substituted with fluorine, chlorine, methoxy or ethoxy, for dimethylamino, or for cyclopropyl optionally substituted with fluorine, chlorine or methyl,
R² stands for hydrogen, chlorine, bromine, for methyl, ethyl, n- or i-propyl, n-, i- , or s-butyl, methoxy, ethoxy, n- or i-propoxy, methylthio, ethylthio, n- or i- propylthio, methylamino, ethylamino, n- or i-propylamino, dimethylamino, diethylamino, ethenyl, propenyl, butenyl, ethinyl, propinyl, butinyl, propenyloxy, butenyloxy, propinyloxy, butinyloxy, propenylthio, butenylthio, propinylthio, butinylthio, propenylamino, butenylamino, propinylamino or butinylamino, in each instance optionally substituted with fluorine, chlorine, methoxy or ethoxy, or for cyclopropyl, cyclobutyl, cyclopentyl, cyclohexyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopropylmethylthio, cyclobutylmethylthio, cyclopentylmethylthio, cyclohexylmethylthio, cyclopropylmethylamino, cyclobutylmethylamino, cyclopentylmethlyamino or cyclohexylmethylamino, in each instance optionally substituted with fluorine, chlorine or methyl,
R³ stands for nitro, cyano, fluorine, chlorine, bromine, for methyl, ethyl, n- or i- propyl, methoxy, ethoxy, n- or i-propoxy, methoxycarbonyl, ethoxycarbonyl, n- or i-propoxycarbonyl, methylthio, ethylthio, n- or i-propylthio, methylsulphinyl, ethylsulphinyl, methylsulphonyl or ethylsulphonyl, in each instance optionally substituted with fluorine, chlorine, methoxy or ethoxy, for cyclopropyl, cyclobutyl, cyclopentyl, cyclohexyl, cyclopropyloxy, cyclobutyloxy, cyclopentyloxy, cyclohexyloxy, cyclopropylthio, cyclobutylthio, cyclopentylthio or cyclohexylthio, in each instance optionally substituted with fluorine, chlorine or methyl, or for phenyl, phenoxy, phenylthio, phenylsulphinyl or phenylsulphonyl, in each instance optionally substituted with cyano, nitro, fluorine, chlorine, bromine, methyl, ethyl, n- or i-propyl, n-, i-, s- or t-butyl, trifluoromethyl, methoxy, ethoxy, n- or i-propoxy, difluoromethoxy or trifluoromethoxy, and
R⁴ stands for hydrogen, nitro, cyano, fluorine, chlorine, bromine, or for methyl, ethyl, n- or i-propyl, methoxy, ethoxy, n- or i-propoxy, methoxycarbonyl, ethoxycarbonyl, n- or i-propoxycarbonyl, methylthio, ethylthio, n- or i- propylthio, methylsulphinyl, ethylsulphinyl, methylsulphonyl or ethylsulphonyl, in each instance optionally substituted with fluorine, chlorine, methoxy or ethoxy.

4. Composition according to claim 1, **characterized in that**
R¹ stands for hydrogen, for methyl, ethyl, ethenyl, methoxy, ethoxy, methylamino or ethylamino, in each instance optionally substituted with fluorine, chlorine, methoxy or ethoxy, for dimethylamino, or for cyclopropyl, optionally substituted with fluorine, chlorine or methyl,
R² stands for hydrogen, chlorine, bromine, for methyl, ethyl, n- or i-propyl, n-, i- or s-butyl, methoxy, ethoxy, n- or i-propoxy, methylthio, ethylthio, n- or i- propylthio, methylamino, ethylamino, n- or i-propylamino, dimethylamino, diethylamino, ethenyl, propenyl, butenyl, ethinyl, propinyl, butinyl, propenyloxy, butenyloxy, propinyloxy, butinyloxy, propenylthio, butenylthio, propinylthio, butinylthio, in each instance optionally substituted with fluorine, chlorine, methoxy or ethoxy, or for cyclopropyl, cyclopropyloxy, cyclopropylthio, cyclopropylmethyl, cyclopropylmethoxy or cyclopropylmethylthio, in each instance optionally substituted with fluorine, chlorine or methyl,
R³ stands for nitro, cyano, fluorine, chlorine, bromine, for methyl, ethyl, n- or i- propyl, methoxy, ethoxy, n- or i-propoxy, methoxycarbonyl, ethoxycarbonyl, n- or i-propoxycarbonyl, methylthio, ethylthio, n- or i-propylthio, methylsulphinyl, ethylsulphinyl, methylsulphonyl or ethylsulphonyl, in each instance optionally substituted with fluorine, chlorine, methoxy or ethoxy, or for phenyl or phenoxy, in each instance optionally substituted with cyano, nitro, fluorine, chlorine, bromine, methyl, ethyl, trifluoromethyl, methoxy, ethoxy, difluoromethoxy or trifluoromethoxy, and
R⁴ stands for hydrogen, nitro, cyano, fluorine, chlorine, bromine, or for methyl, ethyl, n- or i-propyl, methoxy, ethoxy, n- or i-propoxy, methoxycarbonyl, ethoxycarbonyl, n- or i-propoxycarbonyl, methylthio, ethylthio, n- or i- propylthio, methylsulphinyl, ethylsulphinyl, methylsulphonyl or ethylsulphonyl, in each instance optionally substituted with fluorine, chlorine, methoxy or ethoxy.

5. Composition according to claim 1, **characterized in that**
m stands for the numbers 0, 1, 2, 3 or 4,
n stands for the numbers 0, 1, 2 or 3,
X stands for nitro, cyano, carboxy, carbamoyl, formyl, sulfamoyl, hydroxyl, amino, halogen, for methyl, ethyl, n- or i-propyl, n-, i-, s- or t-butyl, methoxy, ethoxy, n- or i-propoxy, n-, i-, s- or t-butoxy, methoxycarbonyl, ethoxycarbonyl, n- or i-propoxycarbonyl, n-, i-, s- or t-butoxycarbonyl, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, methylamino, ethylamino, n- or i-propylamino, n-, i-, s- or t- butylamino, methylaminocarbonyl, ethylaminocarbonyl, n- or i- propylaminocarbonyl, in each instance optionally substituted with cyano, halogen, methoxy, ethoxy, n- or i-propoxy, or for dimethylamino, diethylamino, dimethylaminocarbonyl, or dimethylaminosulphonyl,
Y stands for nitro, cyano, carboxy, carbamoyl, formyl, sulfamoyl, hydroxyl, amino, halogen, or for methyl, ethyl, n- or i-propyl, n, i-, s- or t-butyl, methoxy, ethoxy, n- or i-propoxy, n, i-, s- or t-butoxy, in each instance optionally substituted with cyano, halogen, methoxy, ethoxy, n- or i- propoxy, and
Z stands for the following group 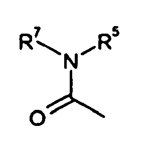 wherein
R⁵ stands for hydrogen, for methyl, ethyl, n- or i-propyl, n-, i-, s- or t-butyl, n-, i-, s- or t-pentyl or neo-pentyl, in each instance optionally substituted with cyano, halogen, methoxy, ethoxy, n- or i-propoxy, n, i-, s- or t-butoxy, for propenyl, butenyl, pentenyl, propinyl, butinyl or pentinyl, in each instance optionally substituted with halogen, or for cyclopropyl, cyclobutyl, cyclopentyl, cyclohexyl, cyclopropylmethyl, cyclobutylmethyl, cyclopentylmethyl or cyclohexylmethyl, in each instance optionally substituted with halogen, methyl, ethyl, n- or i-propyl,
R⁷ stands for hydrogen, for methyl, ethyl, n- or i-propyl, n-, i-, s- or t-butyl, n-, i-, s- or t-pentyl or neo-pentyl, in each instance optionally substituted with cyano, halogen, methoxy, ethoxy, n- or i-propoxy, n, i-, s- or t-butoxy, for methoxy, ethoxy, n- or i-propoxy, n, i-, s- or t-butoxy, for propenyl, butenyl, pentenyl, propinyl, butinyl or pentinyl, in each instance optionally substituted with halogen, for propenyloxy, butenyloxy or pentenyloxy, for cyclopropyl, cyclobutyl, cyclopentyl, cyclohexyl, cyclopropylmethyl, cyclobutylmethyl, cyclopentylmethyl or cyclohexylmethyl, in each instance optionally substituted with halogen, methyl, ethyl, n- or i-propyl, or for phenyl, naphthyl, benzyl or phenylethyl, in each instance optionally substituted with nitro, cyano, halogen, methyl, ethyl, n- or i-propyl, n-, i-, s- or t-butyl, trifluoromethyl, methoxy, ethoxy, n- or i-propoxy, difluoromethoxy or trifluoromethoxy, or, together with R⁵ for butan-1,4-diyl (tetramethylen), 1-oxa-butan-1,4-diyl, pentan-1,5-diyl, 1-oxa-pentan-1,5-diyl or 3-oxa- pentan-1,5-diyl, in each instance optionally substituted with methyl or ethyl.

6. Composition according to claim 1, **characterized in that**
m stands for the numbers 1 or 2,
n stands for the number 0,
X stands for nitro, cyano, fluorine, chlorine, bromine, for methyl, ethyl, n- or i- propyl, methoxy, ethoxy, n- or i-propoxy, methoxycarbonyl, ethoxycarbonyl, n- or i-propoxycarbonyl, methylthio, ethylthio, n- or i-propylthio, methylsulphinyl, ethylsulphinyl, methylsulphonyl, ethylsulphonyl, methylamino, ethylamino, n- or i-propylamino, methylaminocarbonyl, ethylaminocarbonyl, n- or i-propylaminocarbonyl, in each instance optionally substituted with fluorine, chlorine, methoxy or ethoxy, or for dimethylamino, dimethylaminocarbonyl or dimethylaminosulphonyl, and
Z stands for the following group 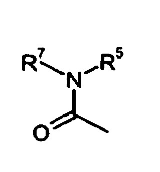 wherein
R⁵ stands for hydrogen, for methyl, ethyl, n- or i-propyl, n- or i-butyl, in each instance optionally substituted with cyano, fluorine, chlorine, methoxy, ethoxy, n- or i-propoxy, for propenyl, butenyl, propinyl or butinyl, in each instance optionally substituted with fluorine and/or chlorine, or for cyclopropyl, cyclobutyl, cyclopentyl, cyclohexyl, cyclopropylmethyl, cyclobutylmethyl, cyclopentylmethyl or cyclohexylmethyl, in each instance optionally substituted with fluorine, chlorine, methyl or ethyl, and
R⁷ stands for hydrogen, for methyl, ethyl, n- or i-propyl, n- i-, s- or t-butyl, n-, i-, s- or t-pentyl or neo-pentyl, in each instance optionally substituted with cyano, fluorine, chlorine, methoxy, ethoxy, n- or i-propoxy, for methoxy, ethoxy, n-or i-propoxy, n-, i-, s- or t-butoxy, for propenyl, butenyl, pentenyl, propinyl, butinyl or pentinyl, in each instance optionally substituted with fluorine and/or chlorine, for propenyloxy, butenyloxy or pentenyloxy, for cyclopropyl, cyclobutyl, cyclopentyl, cyclohexyl, cyclopropylmethyl, cyclobutylmethyl, cyclopentylmethyl or cyclohexylmethyl, in each instance optionally substituted with fluorine, chlorine, methyl or ethyl, or for phenyl, benzyl or phenylethyl, in each instance optionally substituted with nitro, cyano, fluorine, chlorine, bromine, methyl, ethyl, n- or i-propyl, n- i-, s- or t- butyl, trifluoromethyl, methoxy, ethoxy, n- or i-propoxy, difluoromethoxy or trifluoromethoxy, or, together with R⁵, for butan-1,4-diyl (tetramethylen), 1- oxa-butan-1,4-diyl, pentan-1,5-diyl, 1-oxa-pentan-1,5-diyl or 3-oxa-pentan- 1,5-diyl, in each instance optionally substituted with methyl or ethyl.

7. Composition according to any one of claims 1 to 6, **characterized in that** the compound of Formula (I) is one or more compounds selected from compounds 2-(2-methoxycarbonyl-phenylsulphonylaminocarbonyl)-4-methyl-5-n-propoxy-2,4-dihydro-3H-1,2,4-triazole-3-on (propoxycarbazone), 2-(2-trifluoromethoxy-phenylsulphonylaminocarbonyl)-4-methyl-5-methoxy-2,4-dihydro-3H-1,2,4-triazole-3-on (Flucarbazone) and the corresponding sodium salts (propoxycarbazone-sodium and flucarbazone-sodium, respectively).

8. Use of a composition according to any one of claims 1 to 7 for combating undesired plants.

9. A method for combating undesired plants, **characterized in that** the composition according to any one of claims 1 to 7 is allowed to act on the undesired plants and/or their biotope.

10. A method for producing a herbicidal composition, **characterized in that** a composition according to any one of claims 1 to 7 is mixed with surface-active agents and/or extending agents.

## Revendications

1. Agent, **caractérisé par** une teneur efficace en une combinaison de substances actives comprenant
(a) une ou plusieurs arylsulfonylaminocarbonyl-triazolinones substituées de formule (I) 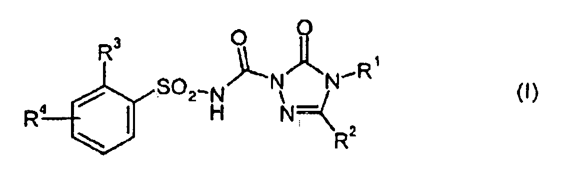 dans laquelle
R¹ représente un atome d'hydrogène, un groupe hydroxy, amino, alkylidènamino en C₂-C₆ ; un groupe alkyle, alcényle, alcynyle, alcoxy, alcényloxy, alkylamino ou dialkylamino, substitué respectivement le cas échéant par un groupe cyano, un atome d'halogène ou un groupe alcoxy en C₁-C₄ et comprenant respectivement jusqu'à 6 atomes de carbone ; un groupe cycloalkyle, cycloalkylalkyle ou cycloalkylamino, substitué respectivement le cas échéant par un groupe cyano, un atome d'halogène ou un groupe alkyle en C₁-C₄ et comprenant respectivement de 3 à 6 atomes de carbone dans les groupes cycloalkyles et le cas échéant de 1 à 4 atomes de carbone dans la partie alkyle ; ou un groupe phényle ou phénylalkyle en C₁-C₄ substitué respectivement le cas échéant par un groupe cyano, nitro, un atome d'halogène, un groupe alkyle en C₁-C₄, halogénoalkyle en C₁-C₄, alcoxy en C₁-C₄ ou halogénoalcoxy en C₁-C₄,
R² représente un atome d'hydrogène, un groupe hydroxy, mercapto, amino, cyano, un atome d'halogène ; un groupe alkyle, alcoxy, alkylthio, alkylamino, dialkylamino, alcényle, alcynyle, alcényloxy, alcynyloxy, alcénylthio, alcynylthio, alcénylamino ou alcynylamino, substitué respectivement le cas échéant par un groupe cyano, un atome d'halogène ou un groupe alcoxy en C₁-C₄ et comprenant respectivement jusqu'à 6 atomes de carbone ; un groupe cycloalkyle, cycloalkyloxy, cycloalkylthio, cycloalkylamino, cycloalkylalkyle, cycloalkylalcoxy, cycloalkylalkylthio ou cycloalkylalkylamino, substitué respectivement le cas échéant par un groupe cyano, un atome d'halogène ou un groupe alkyle en C₁-C₄ et comprenant respectivement de 3 à 6 atomes de carbone dans les groupes cycloalkyles et le cas échéant de 1 à 4 atomes de carbone dans la partie alkyle ; ou un groupe phényle, phénoxy, phénylthio, phénylamino ou phénylalkyle en C₁-C₄ substitué respectivement le cas échéant par un groupe cyano, nitro, un atome d'halogène, un groupe alkyle en C₁-C₄, halogénoalkyle en C₁-C₄, alcoxy en C₁-C₄ ou halogénoalcoxy en C₁-C₄,
R³ représente un groupe nitro, cyano, un atome d'halogène ; un groupe alkyle, alkylcarbonyle, alcoxy, alcoxycarbonyle, alkylthio, alkylsulfinyle, alkylsulfonyle, alkylamino, alcényle, alcényloxy, alcénylthio, alcénylamino, alcynyle, alcynyloxy, alcynylthio, substitué respectivement le cas échéant par un groupe cyano, un atome d'halogène ou un groupe alcoxy en C₁-C₄ et comprenant respectivement jusqu'à 6 atomes de carbone ; un groupe cycloalkyle, cycloalkyloxy, cycloalkylthio, cycloalkylamino, substitué respectivement le cas échéant par un groupe cyano, un atome d'halogène ou un groupe alkyle en C₁-C₄ et comprenant respectivement de 3 à 6 atomes de carbone dans les groupes cycloalkyles ; ou un groupe phényle, phénoxy, phénylthio, phénylsulfinyle, phénylsulfonyle ou phénylamino, substitué respectivement le cas échéant par un groupe cyano, nitro, un atome d'halogène, un groupe alkyle en C₁-C₄, halogénoalkyle en C₁-C₄, alcoxy en C₁-C₄ ou halogénoalcoxy en C₁-C₄, et
R⁴ représente un atome d'hydrogène, un groupe nitro, cyano, un atome d'halogène ; un groupe alkyle, alkylcarbonyle, alcoxy, alcoxycarbonyle, alkylthio, alkylsulfinyle, alkylsulfonyle, alkylamino, alcényle, alcényloxy, alcénylthio, alcénylamino, alcynyle, alcynyloxy ou alcynylthio, substitué respectivement le cas échéant par un groupe cyano, un atome d'halogène ou un groupe alcoxy en C₁-C₄ et comprenant respectivement jusqu'à 6 atomes de carbone ; un groupe cycloalkyle, cycloalkyloxy, cycloalkylthio, cycloalkylamino, substitué respectivement le cas échéant par un groupe cyano, un atome d'halogène ou un groupe alkyle en C₁-C₄ et comprenant respectivement de 3 à 6 atomes de carbone dans les groupes cycloalkyles ; ou un groupe phényle, phénoxy, phénylthio, phénylsulfinyle, phénylsulfonyle ou phénylamino, substitué respectivement le cas échéant par un groupe cyano, nitro, un atome d'halogène, un groupe alkyle en C₁-C₄, halogénoalkyle en C₁-C₄, alcoxy en C₁-C₄ ou halogénoalcoxy en C₁-C₄,
- y compris toutes les formes tautomères possibles des composés de formule générale (I) ainsi que les sels formés à partir des composés de formule générale (I) et des composés basiques -
(« composés efficaces du groupe 1 »)
et
(b) au moins un composé améliorant la tolérance des plantes cultivées provenant du groupe de composés de formule générale (II) suivant 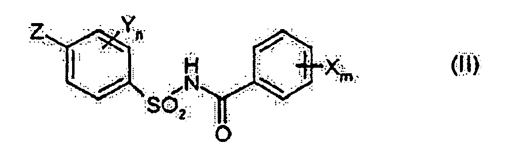 dans laquelle
m représente les chiffres 0 à 5,
n représente les chiffres 0 à 4,
X représente un groupe nitro, cyano, carboxy, carbamoyle, formyle, sulfamoyle, hydroxy, amino, un atome d'halogène ; ou un groupe alkyle, alcoxy, alcoxycarbonyle, alkylthio, alkylsulfinyle, alkylsulfonyle, alkylamino, alkylaminocarbonyle, substitué respectivement le cas échéant par un groupe cyano, un atome d'halogène ou un groupe alcoxy en C₁-C₄ et comprenant respectivement de 1 à 6 atomes de carbone dans les groupes alkyles ; ou un groupe dialkylamino, dialkylaminocarbonyle ou dialkylaminosulfonyle comprenant respectivement de 1 à 6 atomes de carbone dans les groupes alkyles,
Y représente un groupe nitro, cyano, carboxy, carbamoyle, formyle, sulfamoyle, hydroxy, amino, un atome d'halogène ; ou un groupe alkyle ou alcoxy, substitué respectivement le cas échéant par un groupe cyano, un atome d'halogène ou un groupe alcoxy en C₁- C₄ et comprenant respectivement de 1 à 6 atomes de carbone, et
Z représente le groupement suivant : 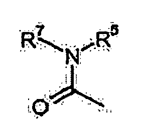 dans lequel
R⁵ représente un atome d'hydrogène ; un groupe alkyle substitué le cas échéant par un groupe cyano, un atome d'halogène ou un groupe alcoxy en C₁-C₄ et comprenant de 1 à 6 atomes de carbone ; un groupe alcényle ou alcynyle substitué respectivement le cas échéant par un atome d'halogène et comprenant respectivement de 3 à 6 atomes de carbone ; ou un groupe cycloalkyle ou cycloalkylalkyle , substitué respectivement le cas échéant par un atome d'halogène ou un groupe alkyle en C₁-C₄ et comprenant respectivement de 3 à 6 atomes de carbone dans le groupe cycloalkyle et le cas échéant de 1 à 4 atomes de carbone dans la partie alkyle, et
R⁷ représente un atome d'hydrogène ; un groupe alkyle substitué le cas échéant par un groupe cyano, un atome d'halogène ou un groupe alcoxy en C₁-C₄ et comprenant de 1 à 6 atomes de carbone ; un groupe alcoxy comprenant de 1 à 6 atomes de carbone ; un groupe alcényle ou alcynyle, substitué respectivement le cas échéant par un atome d'halogène et comprenant respectivement de 3 à 6 atomes de carbone ; un groupe alcényloxy comprenant de 3 à 6 atomes de carbone ; un groupe cycloalkyle ou cycloalkylalkyle, substitué respectivement le cas échéant par un atome d'halogène ou un groupe alkyle en C₁-C₄ et comprenant respectivement de 3 à 6 atomes de carbone dans le groupe cycloalkyle et le cas échéant de 1 à 4 atomes de carbone dans la partie alkyle ; ou un groupe aryle ou arylalkyle substitué respectivement le cas échéant par un groupe nitro, cyano, un atome d'halogène, un groupe alkyle en C₁-C₄, halogénoalkyle en C₁-C₄, alcoxy en C₁-C₄ ou halogénoalcoxy en C₁-C₄ et comprenant respectivement de 6 à 10 atomes de carbone dans le groupe aryle et le cas échéant de 1 à 4 atomes de carbone dans la partie alkyle ; ou bien représente, conjointement avec R⁵, un groupe alcanediyle comprenant de 2 à 6 atomes de carbone ou oxaalcanediyle comprenant de 2 à 5 atomes de carbone, substitué respectivement le cas échéant par un groupe alkyle en C₁-C₄,
(« composés efficaces du groupe 2 »).

2. Agent selon la revendication 1, **caractérisé en ce que**
R¹ représente un atome d'hydrogène, un groupe amino, propylidènamino, butylidènamino ; un groupe méthyle, éthyle, n- ou i-propyle, n-, i-, s- ou t-butyle, éthényle, propényle, butényle, éthynyle, propynyle, butynyle, méthoxy, éthoxy, n- ou i-propoxy, n-, i-, s- ou t-butoxy, propényloxy, butényloxy, méthylamino, éthylamino, n- ou i-propylamino, n-, i-, s- ou t-butylamino, diméthylamino ou diéthylamino, substitué respectivement le cas échéant par un groupe cyano, un atome de fluor, de chlore, un groupe méthoxy ou éthoxy ; un groupe cyclopropyle, cyclobutyle, cyclopentyle, cyclohexyle, cyclopropylméthyle, cyclobutylméthyle, cyclopentylméthyle, cyclohexylméthyle, cyclopropylamino, cyclobutylamino, cyclopentylamino ou cyclohexylamino, substitué respectivement le cas échéant par un groupe cyano, un atome de fluor, de chlore, de brome, un groupe méthyle, éthyle, n- ou i- propyle ; ou un groupe phényle ou benzyle, substitué respectivement le cas échéant par un groupe cyano, nitro, un atome de fluor, de chlore, de brome, un groupe méthyle, éthyle, n- ou i-propyle, n-, i-, s- ou t-butyle, trifluorométhyle, méthoxy, éthoxy, difluorométhoxy ou trifluorométhoxy,
R² représente un atome d'hydrogène, un groupe hydroxy, mercapto, amino, cyano, un atome de fluor, de chlore, de brome ; un groupe méthyle, éthyle, n- ou i-propyle, n-, i-, s- ou t-butyle, méthoxy, éthoxy, n- ou i-propoxy, n-, i-, s- ou t-butoxy, méthylthio, éthylthio, n- ou i-propylthio, n-, i-, s- ou t-butylthio, méthylamino, éthylamino, n- ou i-propylamino, n-, i-, s- ou t- butylamino, diméthylamino, diéthylamino, éthényle, propényle, butényle, éthynyle, propynyle, butynyle, propényloxy, butényloxy, propynyloxy, butynyloxy, propénylthio, buténylthio, propynylthio, butynylthio, propénylamino, buténylamino, propynylamino ou butynylamino, substitué respectivement le cas échéant par un groupe cyano, un atome de fluor, de chlore, un groupe méthoxy ou éthoxy ; un groupe cyclopropyle, cyclobutyle, cyclopentyle, cyclohexyle, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylméthyle, cyclobutylméthyle, cyclopentylméthyle, cyclohexylméthyle, cyclopropylméthoxy, cyclobutylméthoxy, cyclopentylméthoxy, cyclohexylméthoxy, cyclopropylméthylthio, cyclobutylméthylthio, cyclopentylméthylthio, cyclohexylméthylthio, cyclopropylméthylamino, cyclobutylméthylamino, cyclopentylméthylamino ou cyclohexylméthylamino, substitué respectivement le cas échéant par un groupe cyano, un atome de fluor, de chlore, de brome, un groupe méthyle, éthyle, n- ou i- propyle ; ou un groupe phényle, phénoxy, phénylthio, phénylamino ou benzyle, substitué respectivement le cas échéant par un groupe cyano, nitro, un atome de fluor, de chlore, de brome, un groupe méthyle, éthyle, n- ou i-propyle, n-, i-, s- ou t-butyle, trifluorométhyle, méthoxy, éthoxy, difluorométhoxy ou trifluorométhoxy,
R³ représente un groupe nitro, cyano, un atome de fluor, de chlore, de brome ; un groupe méthyle, éthyle, n- ou i-propyle, n-, i-, s- ou t-butyle, acétyle, propionyle, n- ou i-butyroyle, méthoxy, éthoxy, n- ou i-propoxy, n-, i-, s- ou t-butoxy, méthoxycarbonyle, éthoxycarbonyle, n- ou i-propoxycarbonyle, méthylthio, éthylthio, n- ou i-propylthio, n-, i-, s- ou t-butylthio, méthylsulfinyle, éthylsulfinyle, n- ou i-propylsulfinyle, méthylsulfonyle, éthylsulfonyle, n- ou i-propylsulfonyle, méthylamino, éthylamino, n- ou i-propylamino, n-, i-, s- ou t- butylamino, éthényle, propényle, butényle, propényloxy, butényloxy, propénylthio, buténylthio, propénylamino, buténylamino, éthynyle, propynyle, butynyle, propynyloxy, butynyloxy, propynylthio ou butynylthio, substitué respectivement le cas échéant par un groupe cyano, un atome de fluor, de chlore, un groupe méthoxy ou éthoxy; un groupe cyclopropyle, cyclobutyle, cyclopentyle, cyclohexyle, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino ou cyclohexylamino, substitué respectivement le cas échéant par un groupe cyano, un atome de fluor, de chlore, de brome, un groupe méthyle, éthyle, n- ou i- propyle ; ou un groupe phényle, phénoxy, phénylthio, phénylsulfinyle, phénylsulfonyle ou phénylamino, substitué respectivement le cas échéant par un groupe cyano, nitro, un atome de fluor, de chlore, de brome, un groupe méthyle, éthyle, n- ou i-propyle, n-, i-, s- ou t-butyle, trifluorométhyle, méthoxy, éthoxy, n- ou i-propoxy, n-, i-, s- ou t-butoxy, difluorométhoxy ou trifluorométhoxy, et
R⁴ représente un atome d'hydrogène, un groupe nitro, cyano, un atome de fluor, de chlore, de brome ; un groupe méthyle, éthyle, n- ou i-propyle, n-, i-, s- ou t-butyle, acétyle, propionyle, n- ou i-butyroyle, méthoxy, éthoxy, n- ou i-propoxy, n-, i-, s- ou t- butoxy, méthoxycarbonyle, éthoxycarbonyle, n- ou i- propoxycarbonyle, méthylthio, éthylthio, n- ou i-propylthio, n-, i-, s- ou t-butylthio, méthylsulfinyle, éthylsulfinyle, n- ou i- propylsulfinyle, méthylsulfonyle, éthylsulfonyle, n- ou i- propylsulfonyle, méthylamino, éthylamino, n- ou i-propylamino, n-, i-, s- ou t-butylamino, éthényle, propényle, butényle, propényloxy, butényloxy, propénylthio, buténylthio, propénylamino, buténylamino, éthynyle, propynyle, butynyle, propynyloxy, butynyloxy, propynylthio ou butynylthio, substitué respectivement le cas échéant par un groupe cyano, un atome de fluor, de chlore, un groupe méthoxy ou éthoxy ; un groupe cyclopropyle, cyclobutyle, cyclopentyle, cyclohexyle, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino ou cyclohexylamino, substitué respectivement le cas échéant par un groupe cyano, un atome de fluor, de chlore, de brome, un groupe méthyle, éthyle, n- ou i-propyle ; ou un groupe phényle, phénoxy, phénylthio, phénylsulfinyle, phénylsulfonyle ou phénylamino, substitué respectivement le cas échéant par un groupe cyano, nitro, un atome de fluor, de chlore, de brome, un groupe méthyle, éthyle, n- ou i-propyle, trifluorométhyle, méthoxy, éthoxy, n- ou i-propoxy, n-, i-, s- ou t-butoxy, difluorométhoxy ou trifluorométhoxy.

3. Agent selon la revendication 1, **caractérisé en ce que**
R¹ représente un atome d'hydrogène, un groupe amino ; un groupe méthyle, éthyle, éthényle, propényle, éthynyle, propynyle, méthoxy, éthoxy, n- ou i-propoxy, méthylamino ou éthylamino, substitué respectivement le cas échéant par un atome de fluor, de chlore, un groupe méthoxy ou éthoxy ; un groupe diméthylamino ; ou un groupe cyclopropyle, substitué le cas échéant par un atome de fluor, de chlore ou un groupe méthyle,
R² représente un atome d'hydrogène, un atome de chlore, de brome ; un groupe méthyle, éthyle, n- ou i-propyle, n-, i- ou s-butyle, méthoxy, éthoxy, n- ou i-propoxy, méthylthio, éthylthio, n- ou i-propylthio, méthylamino, éthylamino, n- ou i-propylamino, diméthylamino, diéthylamino, éthényle, propényle, butényle, éthynyle, propynyle, butynyle, propényloxy, butényloxy, propynyloxy, butynyloxy, propénylthio, butènylthio, propynylthio, butynylthio, propénylamino, butènylamino, propynylamino ou butynylamino, substitué respectivement le cas échéant par un atome de fluor, de chlore, un groupe méthoxy ou éthoxy ; un groupe cyclopropyle, cyclobutyle, cyclopentyle, cyclohexyle, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylméthyle, cyclobutylméthyle, cyclopentylméthyle, cyclohexylméthyle, cyclopropylméthoxy, cyclobutylméthoxy, cyclopentylméthoxy, cyclohexylméthoxy, cyclopropylméthylthio, cyclobutylméthylthio, cyclopentylméthylthio, cyclohexylméthylthio, cyclopropylméthylamino, cyclobutylméthylamino, cyclopentylméthylamino ou cyclohexylméthylamino, substitué respectivement le cas échéant par un atome de fluor, de chlore, ou un groupe méthyle,
R³ représente un groupe nitro, cyano, un atome de fluor, de chlore, de brome ; un groupe méthyle, éthyle, n- ou i-propyle, méthoxy, éthoxy, n- ou i-propoxy, méthoxycarbonyle, éthoxycarbonyle, n- ou i- propoxycarbonyle, méthylthio, éthylthio, n- ou i-propylthio, méthylsulfinyle, éthylsulfinyle, méthylsulfonyle ou éthylsulfonyle, substitué respectivement le cas échéant par un atome de fluor, de chlore, un groupe méthoxy ou éthoxy; un groupe cyclopropyle, cyclobutyle, cyclopentyle, cyclohexyle, cyclopropyloxy, cyclobutyloxy, cyclopentyloxy, cyclohexyloxy, cyclopropylthio, cyclobutylthio, cyclopentylthio ou cyclohexylthio, substitué respectivement le cas échéant par un atome de fluor, de chlore, ou un groupe méthyle ; ou un groupe phényle, phénoxy, phénylthio, phénylsulfinyle ou phénylsulfonyle, substitué respectivement le cas échéant par un groupe cyano, nitro, un atome de fluor, de chlore, de brome, un groupe méthyle, éthyle, n- ou i-propyle, n-, i-, s- ou t-butyle, trifluorométhyle, méthoxy, éthoxy, n- ou i-propoxy, difluorométhoxy ou trifluorométhoxy, et
R⁴ représente un atome d'hydrogène, un groupe nitro, cyano, un atome de fluor, de chlore, de brome ; ou un groupe méthyle, éthyle, n- ou i- propyle, méthoxy, éthoxy, n- ou i-propoxy, méthoxycarbonyle, éthoxycarbonyle, n- ou i-propoxycarbonyle, méthylthio, éthylthio, n- ou i-propylthio, méthylsulfinyle, éthylsulfinyle, méthylsulfonyle ou éthylsulfonyle, substitué respectivement le cas échéant par un atome de fluor, de chlore, un groupe méthoxy ou éthoxy.

4. Agent selon la revendication 1, **caractérisé en ce que**
R¹ représente un atome d'hydrogène ; un groupe méthyle, éthyle, éthényle, méthoxy, éthoxy, méthylamino ou éthylamino, substitué respectivement le cas échéant par un atome de fluor, de chlore, un groupe méthoxy ou éthoxy ; un groupe diméthylamino ; ou un groupe cyclopropyle, substitué le cas échéant par un atome de fluor, de chlore ou un groupe méthyle,
R² représente un atome d'hydrogène, de chlore, de brome ; un groupe méthyle, éthyle, n- ou i-propyle, n-, i- ou s-butyle, méthoxy, éthoxy, n- ou i-propoxy, méthylthio, éthylthio, n- ou i-propylthio, méthylamino, éthylamino, n- ou i-propylamino, diméthylamino, diéthylamino, éthényle, propényle, butényle, éthynyle, propynyle, butynyle, propényloxy, butényloxy, propynyloxy, butynyloxy, propénylthio, buténylthio, propynylthio, butynylthio, substitué respectivement le cas échéant par un atome de fluor, de chlore, un groupe méthoxy ou éthoxy ; ou un groupe cyclopropyle, cyclopropyloxy, cyclopropylthio, cyclopropylméthyle, cyclopropylméthoxy ou cyclopropylméthylthio, substitué respectivement le cas échéant par un atome de fluor, de chlore, ou un groupe méthyle,
R³ représente un groupe nitro, cyano, un atome de fluor, de chlore, de brome ; un groupe méthyle, éthyle, n- ou i-propyle, méthoxy, éthoxy, n- ou i-proppxy, méthoxycarbonyle, éthoxycarbonyle, n- ou i- propoxycarbonyle, méthylthio, éthylthio, n- ou i-propylthio, méthylsulfinyle, éthylsulfinyle, méthylsulfonyle ou éthylsulfonyle, substitué respectivement le cas échéant par un atome de fluor, de chlore, un groupe méthoxy ou éthoxy ; ou un groupe phényle ou phénoxy, substitué respectivement le cas échéant par un groupe cyano, nitro, un atome de fluor, de chlore, de brome, un groupe méthyle, éthyle, trifluorométhyle, méthoxy, éthoxy, difluorométhoxy ou trifluorométhoxy, et
R⁴ représente un atome d'hydrogène, un groupe nitro, cyano, un atome de fluor, de chlore, de brome ; ou un groupe méthyle, éthyle, n- ou i- propyle, méthoxy, éthoxy, n- ou i-propoxy, méthoxycarbonyle, éthoxycarbonyle, n- ou i-propoxycarbonyle, méthylthio, éthylthio, n- ou i-propylthio, méthylsulfinyle, éthylsulfinyle, méthylsulfonyle ou éthylsulfonyle, substitué respectivement le cas échéant par un atome de fluor, de chlore, un groupe méthoxy ou éthoxy.

5. Agent selon la revendication 1, **caractérisé en ce que**
m représente les chiffres 0, 1, 2, 3 ou 4,
n représente les chiffres 0, 1, 2 ou 3,
X représente un groupe nitro, cyano, carboxy, carbamoyle, formyle, sulfamoyle, hydroxy, amino, un atome d'halogène ; un groupe méthyle, éthyle, n- ou i-propyle, n-, i-, s- ou t-butyle, méthoxy, éthoxy, n- ou i- propoxy, n-, i-, s- ou t-butoxy, méthoxycarbonyle, éthoxycarbonyle, n- ou i-propoxycarbonyle, n-, i-, s- ou t-butoxycarbonyle, méthylthio, éthylthio, n- ou i-propylthio, n-, i-, s- ou t-butylthio, méthylsulfinyle, éthylsulfinyle, n- ou i-propylsulfinyle, méthylsulfonyle, éthylsulfonyle, méthylamino, éthylamino, n- ou i-propylamino, n-, i-, s- ou t- butylamino, méthylaminocarbonyle, éthylaminocarbonyle, n- ou i- propylaminocarbonyle, substitué respectivement le cas échéant par un groupe cyano, un atome d'halogène, un groupe méthoxy, éthoxy, n- ou i-propoxy ; ou un groupe diméthylamino, diéthylamino, diméthylaminocarbonyle ou diméthylaminosulfonyle,
Y représente un groupe nitro, cyano, carboxy, carbamoyle, formyle, sulfamoyle, hydroxy, amino, un atome d'halogène ; ou un groupe méthyle, éthyle, n- ou i-propyle, n-, i-, s- ou t-butyle, méthoxy, éthoxy, n- ou i-propoxy, n-, i-, s- ou t-butoxy, substitué respectivement le cas échéant par un groupe cyano, un atome d'halogène ou un groupe méthoxy, éthoxy, n- ou i-propoxy, et
Z représente le groupement suivant : 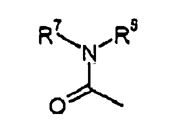 dans lequel
R⁵ représente un atome d'hydrogène ; un groupe méthyle, éthyle, n- ou i-propyle, n-, i-, s- ou t-butyle, n-, i-, s- ou t-pentyle ou néopentyle, substitué respectivement le cas échéant par un groupe cyano, un atome d'halogène, un groupe méthoxy, éthoxy, n- ou i-propoxy, n-, i-, s- ou t-butoxy ; un groupe propényle, butényle, pentényle, propynyle, butynyle ou pentynyle, substitué respectivement le cas échéant par un atome d'halogène ; ou un groupe cyclopropyle, cyclobutyle, cyclopentyle, cyclohexyle, cyclopropylméthyle, cyclobutylméthyle, cyclopentylméthyle ou cyclohexylméthyle, substitué respectivement le cas échéant par un atome d'halogène, un groupe méthyle, éthyle, n- ou i-propyle, et
R⁷ représente un atome d'hydrogène ; un groupe méthyle, éthyle, n- ou i-propyle, n-, i-, s- ou t-butyle, n-, i-, s- ou t-pentyle ou néopentyle, substitué respectivement le cas échéant par un groupe cyano, un atome d'halogène, un groupe méthoxy, éthoxy, n- ou i-propoxy, n-, i-, s- ou t-butoxy ; un groupe méthoxy, éthoxy, n- ou i-propoxy, n-, i-, s- ou t-butoxy ; un groupe propényle, butényle, pentényle, propynyle, butynyle ou pentynyle, substitué respectivement le cas échéant par un atome d'halogène ; un groupe propényloxy, butényloxy ou pentènyloxy ; un groupe cyclopropyle, cyclobutyle, cyclopentyle, cyclohexyle, cyclopropylméthyle, cyclobutylméthyle, cyclopentylméthyle ou cyclohexylméthyle, substitué respectivement le cas échéant par un atome d'halogène, un groupe méthyle, éthyle, n- ou i- propyle ; ou un groupe phényle, naphtyle, benzyle ou phényléthyle, substitué respectivement le cas échéant par un groupe nitro, cyano, un atome d'halogène, un groupe méthyle, éthyle, n- ou i-propyle, n-, i-, s- ou t-butyle, trifluorométhyle, méthoxy, éthoxy, n- ou i-propoxy, difluorométhoxy ou trifluorométhoxy ; ou bien représente, conjointement avec R⁵, un groupe butane-1,4-diyle (tétraméthylène), 1-oxabutane-1,4- diyle, pentane-1,5-diyle, 1-oxapentane-1,5-diyle ou 3- oxapentane-1,5-diyle, substitué respectivement le cas échéant par un groupe méthyle ou éthyle.

6. Agent selon la revendication 1, **caractérisé en ce que**
m représente les chiffres 1 ou 2,
n représente le chiffre 0,
X représente un groupe nitro, cyano, un atome de fluor, de chlore, de brome ; un groupe méthyle, éthyle, n- ou i-propyle, méthoxy, éthoxy, n- ou i-propoxy, méthoxycarbonyle, éthoxycarbonyle, n- ou i- propoxycarbonyle, méthylthio, éthylthio, n- ou i-propylthio, méthylsulfinyle, éthylsulfinyle, méthylsulfonyle, éthylsulfonyle, méthylamino, éthylamino, n- ou i-propylamino, méthylaminocarbonyle, éthylaminocarbonyle, n- ou i-propylaminocarbonyle, substitué respectivement le cas échéant par un atome de fluor, de chlore, un groupe méthoxy ou éthoxy ; ou un groupe diméthylamino, diméthylaminocarbonyle ou diméthylaminosulfonyle, et
Z représente le groupement suivant : 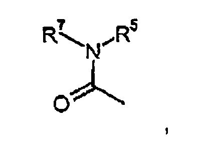 dans lequel
R⁵ représente un atome d'hydrogène ; un groupe méthyle, éthyle, n- ou i- propyle, n- ou i-butyle, substitué respectivement le cas échéant par un groupe cyano, un atome de fluor, de chlore, un groupe méthoxy, éthoxy, n- ou i-propoxy ; un groupe propényle, butényle, propynyle ou butynyle, substitué respectivement le cas échéant par un atome de fluor et/ou de chlore ; ou un groupe cyclopropyle, cyclobutyle, cyclopentyle, cyclohexyle, cyclopropylméthyle, cyclobutylméthyle, cyclopentylméthyle ou cyclohexylméthyle, substitué respectivement le cas échéant par un atome de fluor, de chlore, un groupe méthyle ou éthyle, et
R⁷ représente un atome d'hydrogène ; un groupe méthyle, éthyle, n- ou i- propyle, n-, i-, s- ou t-butyle, n-, i-, s- ou t-pentyle ou néopentyle, substitué respectivement le cas échéant par un groupe cyano, un atome de fluor, de chlore, un groupe méthoxy, éthoxy, n- ou i-propoxy ; un groupe méthoxy, éthoxy, n- ou i-propoxy, n-, i-, s- ou t-butoxy ; un groupe propényle, butényle, pentényle, propynyle, butynyle ou pentynyle, substitué respectivement le cas échéant par un atome de fluor et/ou de chlore ; un groupe propényloxy, butényloxy ou pentènyloxy ; un groupe cyclopropyle, cyclobutyle, cyclopentyle, cyclohexyle, cyclopropylméthyle, cyclobutylméthyle, cyclopentylméthyle ou cyclohexylméthyle, substitué respectivement le cas échéant par un atome de fluor, de chlore, un groupe méthyle ou éthyle ; ou un groupe phényle, benzyle ou phényléthyle, substitué respectivement le cas échéant par un groupe nitro, cyano, un atome de fluor, de chlore, de brome, un groupe méthyle, éthyle, n- ou i-propyle, n-, i-, s- ou t- butyle, trifluorométhyle, méthoxy, éthoxy, n- ou i-propoxy, difluorométhoxy ou trifluorométhoxy ; ou bien représente, conjointement avec R⁵, un groupe butane-1,4-diyle (tétraméthylène), 1-oxabutane- 1,4-diyle, pentane-1,5-diyle, 1-oxapentane-1,5-diyle ou 3- oxapentane-1,5-diyle, substitué respectivement le cas échéant par un groupe méthyle ou éthyle.

7. Agent selon l'une des revendications 1 à 6, **caractérisé en ce que** le composé de formule (I) est un ou plusieurs composés choisis parmi les composés 2-(2-méthoxycarbonyl-phénylsulfonylaminocarbonyl)-4-méthyl-5-n-propoxy-2,4-dihydro-3H-1,2,4-triazol-3-one (propoxycarbazone), 2-(2-trifluorométhoxy-phénylsulfonylaminocarbonyl)-4-méthyl-5-méthoxy-2,4-dihydro-3H-1,2,4-triazol-3-one (flucarbazone) et les sels sodiques correspondants (propoxycarbazone-sodium ou flucarbazone-sodium).

8. Utilisation d'un agent selon l'une des revendications 1 à 7 pour lutter contre les plantes indésirables.

9. Procédé de lutte contre les plantes indésirables, **caractérisé en ce que** l'on laisse agir l'agent selon l'une des revendications 1 à 7 sur les plantes indésirables et/ou sur leur environnement.

10. Procédé de fabrication d'un agent herbicide, **caractérisé en ce que** l'on mélange un agent selon l'une des revendications 1 à 7 avec des tensioactifs et/ou des charges.
